# EUROPEAN PATENT APPLICATION

(11) **EP 4 085 916 A1**
(43) Date of publication of application: **09.11.2022**
(21) Application number: 20892673.3
(22) Date of filing: 25.11.2020
(51) Int. Cl.: A61K 35/74, A61K 35/741, A61K 35/742, A61K 35/744, A61K 35/745, A61K 35/747, A61P 37/06, C12N 1/20, C12N 15/11

(54) **PROPHYLACTIC OR THERAPEUTIC COMPOSITION FOR GRAFT-VERSUS-HOST DISEASE**

(30) Priority: 26.11.2019 JP 2019213436
(71) Applicant: Tokyo Metropolitan Government, Shinjuku-ku Tokyo 163-8001 (JP); Keio University, Tokyo, 108-8345 (JP); JSR Corporation, Tokyo 105-8640 (JP)
(72) Inventor: KAKIHANA Kazuhiko, Tokyo 113-8677 (JP); INAMOTO Kyoko, Tokyo 113-8677 (JP); HONDA Kenya, Tokyo 160-8582 (JP); TAKESHITA Kozue, Tokyo 160-8582 (JP); KIRIDOOSHI Yuuko, Tokyo 105-8640 (JP)
(74) Representative: Mewburn Ellis LLP
(86) International application number: PCT/JP2020/043883
(87) International publication number: WO 2021/106952

(57) **Abstract**

The present invention provides a prophylactic or therapeutic composition for graft-versus-host disease (GVHD).

There is provided a prophylactic or therapeutic composition for GVHD, which comprises bacteria belonging to any genus selected from the group consisting of the following genera: Blautia, Clostridium, unclassified Clostridiales, Actinomyces, Parabacteroides, Lachnoclostridium, Bacteroides, Faecalibacterium, unclassified Lachnospiraceae, Roseburia, Ruminococcus, unclassified Firmicutes, Dorea, Phascolarctobacterium, Sutterella, Megamonas, Collinsella, Eubacterium, and Coprococcus, etc., or any combination of bacteria belonging to these genera.

## Description

### TECHNICAL FIELD

The present invention relates to a composition comprising a microbiota, and more particularly relates to a prophylactic or therapeutic composition for graft-versus-host disease (GVHD).

### BACKGROUND ART

Allogeneic hematopoietic stem cell transplantation (allo-HSCT) has been widely used as a radical therapy for various blood diseases, but acute GVHD associated with allo-HSCT is comparable to recurrence and infection among serious complications. Agents used in initial therapy (primary therapy) for this GVHD are adrenocorticosteroid hormones (steroids), but only about half of them are confirmed to be effective (Blood. 2007; 109(10): 4119-4126. (Non-patent Document 1)), and no secondary therapy has been established.

Enterobacteria and their metabolites have been widely known to play important roles in inflammatory suppression and immunomodulation in the gut, and recent reports have suggested the applicability of fecal microbiota transplantation (FMT) to GVHD following allo-HSCT (Blood. 2014; 124(7): 1174-1182. (Non-patent Document 2)). Moreover, as to FMT, the inventions disclosed in JP 2013-537531 A (Patent Document 1), JP 2016-501852 A (Patent Document 2) and Japanese Patent No. 6408092 have been known.

However, the relation between GVHD and FMT is not clear.

### Prior Art Documents

### Patent Documents

Patent Document 1: JP 2013-537531 A
Patent Document 2: JP 2016-501852 A
Patent Document 3: Japanese Patent No. 6408092

### Non-patent Documents

Non-patent Document 1: Blood. 2007; 109(10): 4119-4126.
Non-patent Document 2: Blood. 2014; 124(7): 1174-1182.
Non-patent Document 3: Bone Marrow Transplantation 1995; 15, 825-828.

### SUMMARY OF THE INVENTION

### PROBLEM TO BE SOLVED BY THE INVENTION

The object of the present invention is to provide a prophylactic or therapeutic composition particularly for acute gut GVHD

### MEANS TO SOLVE THE PROBLEM

As a result of extensive and intensive efforts made to solve the problem stated above, the inventors of the present invention have succeeded in preventing or treating GVHD by transplantation of a composition comprising a feces-derived microbiota, and have identified bacteria effective for the prevention or treatment of GVHD from a feces-derived microbiota, thereby completing the present invention.

Namely, the present invention is as follows.
(1) A prophylactic or therapeutic composition for GVHD, which comprises bacteria belonging to any genus selected from the group consisting of the following genera:
   Blautia,
   Clostridium,
   unclassified Clostridiales,
   Actinomyces,
   Parabacteroides,
   Lachnoclostridium,
   Bacteroides,
   Faecalibacterium,
   unclassified Lachnospiraceae,
   Roseburia,
   Ruminococcus,
   unclassified Firmicutes,
   Dorea,
   Phascolarctobacterium,
   Sutterella,
   Megamonas,
   Collinsella,
   Eubacterium,
   Coprococcus,
   Schaalia,
   Alistipes,
   Bifidobacterium,
   Lactobacillus,
   Veillonella,
   Anaerostipes,
   Bilophila,
   Butyricicoccus,
   Barnesiella,
   Fusicatenibacter,
   Flavonifractor,
   unclassified Ruminococcaceae,
   unclassified Clostridiaceae,
   Faecalicatena,
   Prevotella,
   Megasphaera,
   Robinsoniella,
   Faecalitalea,
   Lachnospira, and
   Romboutsia,
   or any combination of bacteria belonging to these genera.
(2) The composition according to (1) above, which comprises bacteria belonging to any genus selected from the group consisting of the following genera:
   Blautia,
   Clostridium,
   unclassified Clostridiales,
   Actinomyces,
   Parabacteroides,
   Lachnoclostridium,
   Bacteroides,
   Faecalibacterium,
   unclassified Lachnospiraceae,
   Roseburia,
   Ruminococcus,
   unclassified Firmicutes,
   Dorea,
   Phascolarctobacterium,
   Sutterella,
   Megamonas,
   Collinsella,
   Eubacterium, and
   Coprococcus,
   or any combination of bacteria belonging to these genera.
(3) The composition according to (1) above, which comprises bacteria belonging to any genus selected from the group consisting of the following genera:
   Blautia,
   Clostridium,
   unclassified Clostridiales,
   Actinomyces,
   Parabacteroides,
   Lachnoclostridium,
   Bacteroides,
   unclassified Lachnospiraceae,
   Roseburia,
   Ruminococcus,
   Phascolarctobacterium,
   Faecalibacterium,
   Schaalia,
   Alistipes,
   Bifidobacterium,
   Lactobacillus,
   Veillonella,
   Dorea,
   unclassified Firmicutes,
   Anaerostipes,
   Collinsella,
   Butyricicoccus,
   Barnesiella, and
   Fusicatenibacter,
   or any combination of bacteria belonging to these genera.
(4) The composition according to (3) above, which comprises bacteria belonging to any genus selected from the group consisting of the following genera:
   Blautia,
   Clostridium,
   unclassified Clostridiales,
   Actinomyces,
   Parabacteroides,
   Lachnoclostridium, and
   Bacteroides,
   or any combination of bacteria belonging to these genera.
(5) The composition according to (1) above, which comprises bacteria belonging to any genus selected from the group consisting of the following genera:
   Faecalibacterium,
   unclassified Clostridiales,
   unclassified Lachnospiraceae,
   Roseburia,
   Ruminococcus,
   unclassified Firmicutes,
   Dorea,
   Phascolarctobacterium,
   Sutterella,
   Bacteroides,
   Blautia,
   Parabacteroides,
   Megamonas,
   Collinsella,
   Eubacterium,
   Coprococcus,
   Anaerostipes,
   Bilophila,
   Flavonifractor,
   unclassified Ruminococcaceae,
   unclassified Clostridiaceae,
   Butyricicoccus,
   Faecalicatena,
   Prevotella,
   Clostridium,
   Lachnoclostridium,
   Alistipes,
   Megasphaera,
   Robinsoniella,
   Fusicatenibacter,
   Barnesiella,
   Faecalitalea,
   Lachnospira, and
   Romboutsia,
   or any combination of bacteria belonging to these genera.
(6) The composition according to (5) above, which comprises bacteria belonging to any genus selected from the group consisting of the following genera:
   Faecalibacterium,
   unclassified Clostridiales,
   unclassified Lachnospiraceae,
   Roseburia,
   Ruminococcus,
   unclassified Firmicutes,
   Dorea,
   Phascolarctobacterium,
   Sutterella,
   Bacteroides,
   Blautia,
   Parabacteroides,
   Megamonas,
   Collinsella,
   Eubacterium, and
   Coprococcus,
   or any combination of bacteria belonging to these genera.
(7) A prophylactic or therapeutic composition for GVHD, which comprises bacteria comprising DNA consisting of a nucleotide sequence sharing 94% or more homology with any of the nucleotide sequences shown in SEQ ID NOs: 1 to 120, or any combination of these bacteria.
(8) The composition according to (7) above, which comprises bacteria comprising DNA consisting of a nucleotide sequence sharing 94% or more homology with any of the nucleotide sequences shown in SEQ ID NOs: 121 to 152, or any combination of these bacteria.
(9) The composition according to (7) above, which comprises bacteria comprising DNA consisting of a nucleotide sequence sharing 94% or more homology with any of the nucleotide sequences shown in SEQ ID NOs: 153 to 198, or any combination of these bacteria.
(10) The composition according to (9) above, which comprises bacteria comprising DNA consisting of a nucleotide sequence sharing 94% or more homology with any of the nucleotide sequences shown in SEQ ID NOs: 199 to 201, or any combination of these bacteria.
(11) The composition according to (7) above, which comprises bacteria comprising DNA consisting of a nucleotide sequence sharing 94% or more homology with any of the nucleotide sequences shown in SEQ ID NOs: 202 to 312, or any combination of these bacteria.
(12) The composition according to (11) above, which comprises bacteria comprising DNA consisting of a nucleotide sequence sharing 94% or more homology with any of the nucleotide sequences shown in SEQ ID NOs: 313 to 344, or any combination of these bacteria.
(13) The composition according to (1) above, wherein the bacteria comprise at least one selected from the group consisting of:
   Bacteroides vulgatus,
   Bacteroides stercoris,
   Bacteroides uniformis,
   Blautia wexlerae,
   Bacteroides sp. AR29,
   Dorea longicatena,
   Phascolarctobacterium faecium,
   Blautia massiliensis,
   Ruminococcus sp. K-1,
   Bacteroides ovatus,
   Faecalibacterium prausnitzii,
   Megamonas funiformis,
   Bifidobacterium adolescentis,
   [Ruminococcus] torques,
   Collinsella aerofaciens,
   Parabacteroides merdae,
   butyrate-producing bacterium M104/1,
   Bifidobacterium faecale,
   Clostridium sp. 826,
   Bacteroides coprocola,
   Roseburia faecis,
   Lachnospiraceae bacterium DJF_VP18k1,
   Clostridium sp. AT4,
   Lactobacillus rogosae,
   butyrate-producing bacterium A2-207,
   Roseburia sp. 1120,
   Bacteroides dorei,
   Bifidobacterium pseudocatenulatum,
   Megasphaera massiliensis,
   Bacteroides massiliensis,
   Anaerostipes hadrus,
   Alistipes putredinis,
   Parabacteroides sp. D25,
   Roseburia inulinivorans,
   Bilophila sp. 4_1_30,
   Flavonifractor plautii,
   Eubacterium ventriosum,
   Clostridiales bacterium 80/3,
   Butyricicoccus faecihominis,
   Bacteroides cellulosilyticus,
   Coprococcus catus,
   Parabacteroides j ohnsonii,
   Lachnospiraceae bacterium DJF_RP14,
   [Ruminococcus] gnavus,
   Bifidobacterium longum,
   Bacteroides xylanisolvens,
   Prevotella stercorea,
   Bacteroides plebeius,
   Firmicutes bacterium DJF VR50,
   Sutterella wadsworthensis,
   Lachnospiraceae bacterium 1_1_57FAA,
   Blautia obeum,
   Barnesiella intestinihominis,
   [Eubacterium] eligens,
   Coprococcus comes,
   Ruminococcus sp. DJF_VR70k1,
   butyrate-producing bacterium A2-175,
   Faecalitalea cylindroides,
   Blautia sp. YHC-4,
   [Clostridium] glycyrrhizinilyticum,
   Ruminococcus sp. 653,
   Ruminococcus lactaris,
   butyrate-producing bacterium SM6/1,
   butyrate-producing bacterium SS3/4,
   Clostridiaceae bacterium DJF_LS40,
   Bacteroides sp. 1_1_30,
   Bacteroides nordii,
   Fusicatenibacter saccharivorans,
   butyrate-producing bacterium SL7/1,
   [Clostridium] scindens,
   Parabacteroides distasonis,
   Schaalia odontolytica,
   Bacteroides faecis,
   Bacteroides fragilis,
   [Clostridium] bolteae, and
   Clostridium sp. HGF2.
(14) The composition according to (13) above, wherein the bacteria comprise at least one selected from the group consisting of:
   Bacteroides vulgatus,
   Bacteroides stercoris,
   Bacteroides uniformis,
   Blautia wexlerae,
   Bacteroides sp. AR29,
   Dorea longicatena,
   Phascolarctobacterium faecium,
   Blautia massiliensis,
   Ruminococcus sp. K-1,
   Bacteroides ovatus,
   Faecalibacterium prausnitzii,
   Megamonas funiformis,
   Bifidobacterium adolescentis,
   [Ruminococcus] torques,
   Collinsella aerofaciens,
   Parabacteroides merdae,
   butyrate-producing bacterium M104/1,
   Bifidobacterium faecale,
   Clostridium sp. 826,
   Bacteroides coprocola,
   Roseburia faecis,
   Lachnospiraceae bacterium DJF_VP18k1,
   Clostridium sp. AT4,
   Lactobacillus rogosae,
   butyrate-producing bacterium A2-207,
   Roseburia sp. 1120, and
   Bacteroides dorei.
(15) The composition according to (13) above, wherein the bacteria comprise at least one selected from the group consisting of:
   Bacteroides vulgatus,
   Bacteroides stercoris,
   Bacteroides uniformis,
   Blautia wexlerae,
   Bacteroides sp. AR29,
   Dorea longicatena,
   Phascolarctobacterium faecium,
   Blautia massiliensis,
   Ruminococcus sp. K-1, and
   Bacteroides ovatus.
(16) The composition according to any one of (1) to (15) above, wherein the GVHD is steroid-refractory or steroid-dependent GVHD
(17) The composition according to any one of (1) to (15) above, wherein the GVHD is acute gut GVHD
(18) A capsule formulation for the prevention or treatment of GVHD, which comprises the composition according to any one of (1) to (17) above.
(19) A therapeutic method for GVHD, which comprises administering the composition according to any one of (1) to (17) above or the capsule formulation according to (18) above to a GVHD patient.
(20) A prophylactic method for GVHD, which comprises administering the composition according to any one of (1) to (17) above or the capsule formulation according to (18) above either before or after or both before and after hematopoietic stem cell transplantation to a patient who is a subject of the hematopoietic stem cell transplantation.
(21) A method for preparing a suspension for fecal microbiota transplantation, which comprises confirming the presence of the bacteria shown in any one of (1) to (15) above in feces collected from a human subject, and suspending the feces confirmed for the presence of the bacteria into an aqueous medium.
(22) A method for preparing a bacterial mixture for fecal microbiota transplantation, which comprises confirming the presence of the bacteria shown in any one of (1) to (15) above in feces collected from a human subject, separating the bacteria from the feces confirmed for the presence of the bacteria, and mixing the separated bacteria.
(23) A method for preparing a formulation for fecal microbiota transplantation, which comprises confirming the presence of the bacteria shown in any one of (1) to (15) above in feces collected from a human subject, separating the bacteria from the feces confirmed for the presence of the bacteria, and formulating the separated bacteria.
(24) The method according to (23) above, wherein the formulation is a capsule formulation.
(25) The method according to any one of (21) to (24) above, wherein the presence of the bacteria is confirmed by 16S rRNA gene analysis.

### EFFECTS OF THE INVENTION

The present invention enables the prevention or treatment of acute gut GVHD

### BRIEF DESCRIPTION OF THE DRAWINGS

Figure 1 shows the results of α diversity evaluation by the Shannon index.
Figure 2 shows the relative proportions of fecal microbiota at the genus level before and after FMT.
Figure 3 shows the results of microbiota analysis based on the 16S rRNA gene in bacteria belonging to each genus.
Figure 4 shows the results of microbiota analysis based on the 16S rRNA gene in bacteria belonging to each genus.
Figure 5 shows the results of microbiota analysis based on the 16S rRNA gene in bacteria belonging to each genus.
Figure 6 shows the results of microbiota analysis based on the 16S rRNA gene in bacteria belonging to each genus.
Figure 7 shows the results of microbiota analysis based on the 16S rRNA gene in bacteria belonging to each genus.
Figure 8 shows the results of microbiota analysis based on the 16S rRNA gene in bacteria belonging to each genus.
Figure 9 shows the results of microbiota analysis based on the 16S rRNA gene in bacteria belonging to each genus.
Figure 10 shows the results of microbiota analysis based on the 16S rRNA gene in bacteria belonging to the genus Corynebacterium.
Figure 11 shows the results of microbiota analysis based on the 16S rRNA gene in each species.
Figure 12 shows the results of microbiota analysis based on the 16S rRNA gene in each species.
Figure 13 shows the results of microbiota analysis based on the 16S rRNA gene in each species.
Figure 14 shows the results of microbiota analysis based on the 16S rRNA gene in each species.
Figure 15 shows the results of microbiota analysis based on the 16S rRNA gene in each species.
Figure 16 shows the results of microbiota analysis based on the 16S rRNA gene in each species.
Figure 17 shows the results of microbiota analysis based on the 16S rRNA gene in each species.
Figure 18 shows the results of microbiota analysis based on the 16S rRNA gene in each species.
Figure 19 shows the results of microbiota analysis based on the 16S rRNA gene in each species.

### DESCRIPTION OF EMBODIMENTS

In the expectation that intervention in the gut microbiota would lead to novel prophylactic and therapeutic methods for GVHD following hematopoietic stem cell transplantation, the inventors of the present invention have attempted to perform fecal microbiota transplantation (FMT) on GVHD patients. FMT is a therapy designed to administer a fecal suspension from a normal subject into the gut of a patient, thereby allowing high volume administration of a normal microbiota, and this therapy has been attempted for use in diseases which are deemed to be associated with dysbiosis of the gut microbiota.

Further, the inventors of the present invention have performed gut microbiota analysis before and after FMT on patients who had developed GVHD following hematopoietic stem cell transplantation, whereby bacteria useful for the prevention and/or treatment of GVHD have been identified. As a result of the analysis, it has been found that there are clear differences in the distribution of the engrafted microbiota between patients whose GVHD has gotten better and the other patients. Further, the engrafted gut microbiota in patients whose GVHD has gotten better were picked up as bacteria useful for the treatment of GVHD, and then grouped under unique indicators such as heterogeneity in abundance due to the therapeutic effect of FMT in recipients, as analyzed by two group comparison (between CR or PR group and Others group), the length of the engraftment period in recipients, etc.

As a result, the inventors of the present invention have succeeded in identifying bacteria useful for the prevention and/or treatment of GVHD.

### 1. FMT

Since a fecal microbiota is contained in feces per se or a treated product of feces, a treated product of feces can be used as the composition of the present invention. Such a treated product of feces includes not only a suspension of collected feces in an appropriate solvent (e.g., physiological saline, buffer), but also a filtrate of this suspension passed through an appropriate sieve, gauze, filter or the like (e.g., pore size: 0.1 mm to 0.5 mm), or a precipitate of this suspension obtained after centrifugation. Further, these compositions may be frozen in a freezer or with liquid nitrogen, or may be subjected to lyophilization or spray drying. In the above case where feces are prepared into a suspension with a solvent, feces may be suspended with 1 to 20 ml of liquid per gram of feces. In a case where the prepared suspension is centrifuged to isolate bacteria, the bacteria may be suspended again in 0.2 to 1 mL of liquid per gram of bacteria and then provided for use.

For freezing or lyophilization, cryoprotectants and/or lyoprotectants may be added, as exemplified by various sugars (e.g., sucrose, fructose, lactose, mannitol), glycerol, polyethylene glycol (PEG), trehalose, glycine, glucose, dextran, erythritol and so on.

In the present invention, the collected feces or a treated product thereof may be stored for 6 to 10 hours after collection or treatment of the feces. The storage temperature is not limited in any way, but cold storage (e.g., 4°C) is preferred for this purpose.

The thus prepared composition is used as a material for FMT. The prepared FMT material is preferably stored under anaerobic conditions (e.g., in an anaerobic unit, in an anaerobic bag) until use. In this case, cold storage (e.g., 4°C) is also preferred.

In the present invention, a composition comprising a fecal microbiota (i.e., an untreated or treated fecal material) is transplanted between different individuals, for example, between humans or between animals. A composition for use in FMT may be transplanted to the same individual whose feces were collected, or may be configured such that a fecal microbiota collected from one individual is transplanted to another individual.

The disease to be targeted is GVHD, as exemplified by GVHD following hematopoietic stem cell transplantation. GVHD includes, but is not limited to, acute gut GVHD

Transplantation may be performed in any manner, either by oral or parenteral administration. Examples include transplantation via a gastroduodenal tube, internal use in the form of capsules or the like, administration of suppositories, transplantation into the colon through a colon fiberscope or high pressure enema, etc.

The amount used for single transplantation is 150 ml to 300 ml in the case of liquid form, which is given once a day. Depending on the condition of a recipient, transplantation may be repeated every 4 days to 2 weeks, twice to 4 times in total.

In this way, the composition of the present invention enables the treatment of GVHD when transplanted (administered) to GVHD patients.

For assessment of the therapeutic effect, the results of the maximum effect at 4 weeks after the final administration and within the observation period are used to evaluate the response rate (CR + PR), which is the sum of complete response (CR) and partial response (PR). A comparative analysis is made between microbiota in patients (group) assessed as CR or PR and microbiota in patients (group) assessed as the group of others. Aliquots of the donor fecal preparation and the patient's feces (e.g., collected before FMT, and at 1 to 3 days and 1, 2 and 4 weeks after the final FMT) are used for analysis of gut microbiota at each time point.

### 2. Heterogeneity in the abundance of bacteria between CR or PR group and Others group

In the present invention, the following two methods are used for narrow down search based on heterogeneity in the abundance of bacteria between CR or PR group and Others group.

### Method 1. Narrow down search with median

(1) At each time point of fecal collection, the average of relative abundance of the respective genera or OTUs (operational taxonomic units) in samples of the same group is calculated for the CR or PR group (hereinafter referred to as "CR or PR group") and the group of others (hereinafter referred to as "Others group"), and genera or OTUs in which a greater one of the averages of the "CR or PR group" and the "Others group" is 0.1% or more are used for analysis.
(2) At each time point, the median of relative abundance of the genera or OTUs in samples of the same group is calculated for each of the CR or PR group and the Others group.
(3) The median Log2 fold change (CR or PR group/Others group) is calculated. The Log2 fold change intended here refers to a logarithmic value (base 2) of the ratio obtained by dividing the median of the CR or PR group by the median of the Others group. Such a median may include 0; and hence 0.0001 is added to each median before calculation of the Log2 fold change.
(4) Genera or OTUs showing a Log2 fold change of 0.5 or more are assumed to be abundant in the CR or PR group, while those showing a Log2 fold change of -0.5 or less are assumed to be abundant in the Others group. However, genera or OTUs showing a negative Log2 Fold change for the ratio of median between the donor and the recipient before FMT in the CR or PR group, or genera or OTUs for which the carrying rate of bacteria belonging thereto is 0 in the donor are excluded, because they cannot be determined to be transplanted through FMT from the donor.
(5) At the time points of fecal collection after fecal transplantation, the number of times when the Log2 fold change was 0.5 or more or was -0.5 or less is counted.
(6) In step 5, cases where the number of times when the Log2 fold change is 0.5 or more exceeds 1 are assumed to be preferred, and bacteria showing a higher number of times are assessed to give more contribution to the effect of FMT. If the number of times counted exceeds more than half of the number of fecal collection after transplantation, such bacteria are assessed to give more contribution to the effect of FMT. However, if the number of times when the Log2 fold change is -0.5 or less exceeds the number of times when the Log2 fold change is 0.5 or more, such bacteria are excluded because they are not determined to be abundant in the CR or PR group.

### Method 2. Narrow down search for bacteria with mean and carrying rate

(1) At each time point of fecal collection, the average of relative abundance of the respective genera or OTUs in samples of the same group is calculated for the CR or PR group (hereinafter referred to as "CR or PR group") and the group of others (hereinafter referred to as "Others group"), and genera or OTUs in which a greater one of the averages of the "CR or PR group" and the "Others group" is 0.1% or more are used for analysis.
(2) At each time point, the mean of relative abundance of the genera or OTUs in samples of the same group is calculated for each of the CR or PR group and the Others group. Further, at each time point, the carrying rate of genera or OTUs (i.e., the proportion of donors or recipients in which the relative abundance of these genera or OTUs is greater than zero) in each group is calculated.
(3) The mean Log2 fold change (CR or PR group/Others group) is calculated. The Log2 fold change intended here refers to a logarithmic value (base 2) of the ratio obtained by dividing the mean of the CR or PR group by the mean of the Others group. Such a mean may include 0; and hence 0.0001 is added to each mean before calculation of the Log2 fold change.
(4) At the time points of fecal collection after fecal transplantation, the number of times when the Log2 fold change was 0.5 or more and the carrying rate in the CR or PR group was 20% or more is counted. Genera or OTUs showing a higher number of times were assumed to be more abundant in the CR or PR group. However, in (4), genera or OTUs showing a negative Log2 Fold change of the ratio of mean between the donor and the recipient before FMT in the CR or PR group, or genera or OTUs for which the carrying rate of bacteria belonging thereto is 0 in the donor are excluded, because they cannot be determined to be transplanted through FMT from the donor.
(5) At the time points of fecal collection after fecal transplantation, the number of times when the Log2 fold change was -0.5 or less and the carrying rate in the Others group was 20% or more is counted. Genera or OTUs showing a higher number of times are assumed to be more abundant in the Others group.
(6) In (4), cases where the number of times counted exceeds 1 are assumed to be preferred, and bacteria showing a higher number of times are assessed to give more contribution to the effect of FMT. If the number of times counted exceeds more than half of the number of fecal collection after transplantation, such bacteria are assessed to give more contribution to the effect of FMT. However, if the number of times in (5) exceeds the number of times in (4), such bacteria are excluded because they are not determined to be abundant in the CR or PR group.

### 3. Microbiota constituting a composition

### 3-1. Definition by sequence

The bacteria extracted by narrow down search using Methods 1 and 2 are bacteria which have DNA shearing 94% or more homology with any of the nucleotide sequences shown in SEQ ID NOs: 1 to 120 in the sequence of their 16S rRNA gene (e.g., v1-v2 region).

As used herein, the expression "94% or more homology" is intended to mean being, for example, 94% or more, 95% or more, 96% or more, 97% or more, 98% or more, 99% or more, 99.5% or more, 99.8% or more, 99.9% or more, or 100% homologous (the same applies hereinafter).

More preferred are bacteria which have DNA sharing 94% or more homology with any of the nucleotide sequences shown in SEQ ID NOs: 121 to 152.

In another embodiment of the present invention, the bacteria extracted by narrow down search using Methods 1 and 2 are bacteria which have DNA sharing 97% or more homology with any of the nucleotide sequences shown in SEQ ID NOs: 1 to 120 in the sequence of their 16S rRNA gene (e.g., v1-v2 region).

More preferred are bacteria which have DNA sharing 97% or more homology with any of the nucleotide sequences shown in SEQ ID NOs: 121 to 152.

In the present invention, bacteria determined to be preferred by Method 1 (narrow down search with median) are bacteria which have DNA sharing 94% or more homology with any of the nucleotide sequences shown in SEQ ID NOs: 153 to 198 in the sequence of the v1-v2 region in their 16S rRNA gene.

More preferred are bacteria which have DNA sharing 94% or more homology with any of the nucleotide sequences shown in SEQ ID NOs: 199 to 201.

In yet another embodiment of the present invention, bacteria determined to be preferred by Method 1 (narrow down search with median) are bacteria which have DNA sharing 97% or more homology with any of the nucleotide sequences shown in SEQ ID NOs: 153 to 198 in the sequence of the v1-v2 region in their 16S rRNA gene.

More preferred are bacteria which have DNA sharing 97% or more homology with any of the nucleotide sequences shown in SEQ ID NOs: 199 to 201.

In the present invention, bacteria determined to be preferred by Method 2 (narrow down search with mean and carrying rate) are bacteria which have DNA sharing 94% or more homology with any of the nucleotide sequences shown in SEQ ID NOs: 202 to 312 in the sequence of the v1-v2 region in their 16S rRNA gene.

More preferred are bacteria which have DNA sharing 94% or more homology with any of the nucleotide sequences shown in SEQ ID NOs: 313 to 344.

In yet another embodiment of the present invention, bacteria determined to be preferred by Method 2 (narrow down search with mean and carrying rate) are bacteria which have DNA sharing 97% or more homology with any of the nucleotide sequences shown in SEQ ID NOs: 202 to 312 in the sequence of the v1-v2 region in their 16S rRNA gene.

More preferred are bacteria which have DNA sharing 97% or more homology with any of the nucleotide sequences shown in SEQ ID NOs: 313 to 344.

### 3-2. Definition by genus name

In the present invention, bacteria determined to be preferred by Method 1 (narrow down search with median) are those belonging to the genera given below. Bacteria belonging to "unclassified taxonomy names" (e.g., unclassified Clostridiales) are bacteria which have DNA sharing 94% or more homology with the nucleotide sequence of 16S rRNA gene in bacteria found under the species names shown in Table 1A blow.

Blautia
Clostridium
unclassified Clostridiales
Actinomyces
Parabacteroides
Lachnoclostridium
Bacteroides
unclassified Lachnospiraceae
Roseburia
Ruminococcus
Phascolarctobacterium
Faecalibacterium
Schaalia
Alistipes
Bifidobacterium
Lactobacillus
Veillonella
Dorea
unclassified Firmicutes
Anaerostipes
Collinsella
Bilophila
Butyricicoccus
Barnesiella
Fusicatenibacter

### [Table 1A]

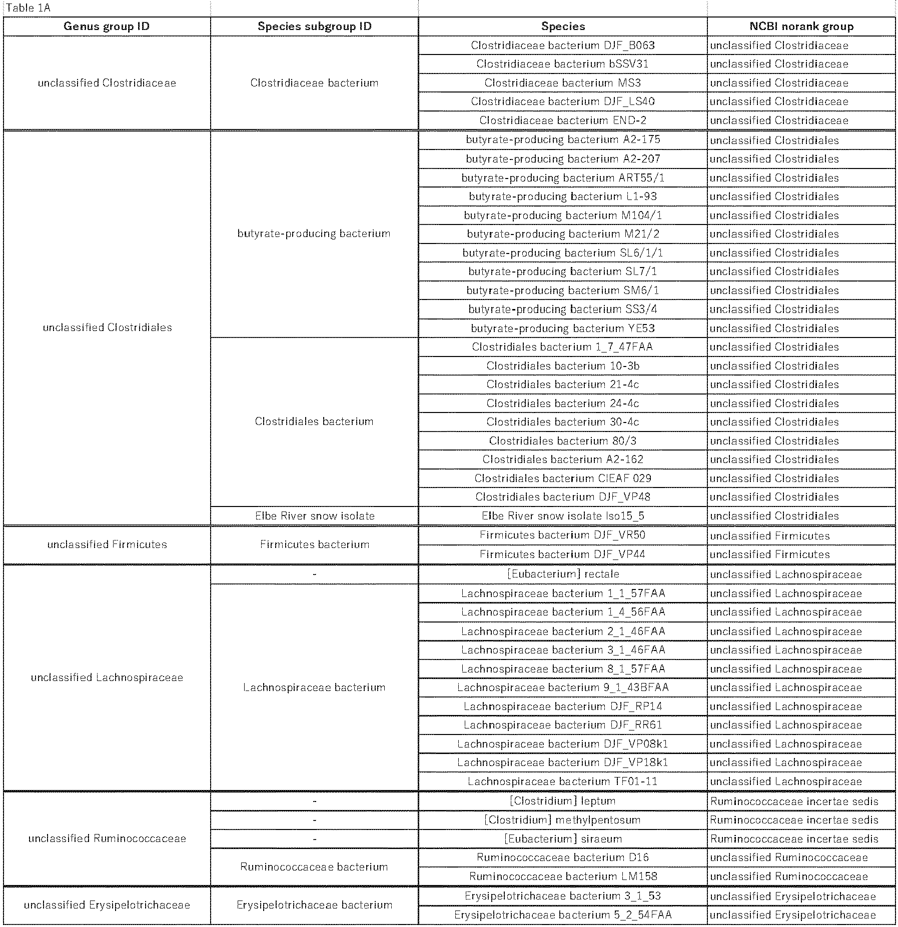

Among those listed above, bacteria determined to be more preferred in terms of the length of the engraftment period in recipients are those belonging to the following genera.
Blautia
Clostridium
unclassified Clostridiales
Actinomyces
Parabacteroides
Lachnoclostridium
Bacteroides

Likewise, in the present invention, bacteria determined to be preferred by Method 2 (narrow down search with mean and carrying rate) are those belonging to the genera given below. Bacteria belonging to "unclassified taxonomy names" are bacteria which have DNA sharing 94% or more homology with the nucleotide sequence of 16S rRNA gene in bacteria found under the species names shown in Table 1A.

Faecalibacterium
unclassified Clostridiales
unclassified Lachnospiraceae
Roseburia
Ruminococcus
unclassified Firmicutes
Dorea
Phascolarctobacterium
Sutterella
Bacteroides
Blautia
Parabacteroides
Megamonas
Collinsella
Eubacterium
Coprococcus
Anaerostipes
Bilophila
Flavonifractor
unclassified Ruminococcaceae
unclassified Clostridiaceae
Butyricicoccus
Faecalicatena
Prevotella
Clostridium
Lachnoclostridium
Alistipes
Megasphaera
Robinsoniella
Fusicatenibacter
Barnesiella
Faecalitalea
Lachnospira
Romboutsia

Among those listed above, bacteria determined to be more preferred in terms of the length of the engraftment period in recipients are those belonging to the following genera.
Faecalibacterium
unclassified Clostridiales
unclassified Lachnospiraceae
Roseburia
Ruminococcus
unclassified Firmicutes
Dorea
Phascolarctobacterium
Sutterella
Bacteroides
Blautia
Parabacteroides
Megamonas
Collinsella
Eubacterium
Coprococcus

The genera of bacteria extracted by the above two methods for narrow down search are as listed below. Bacteria belonging to "unclassified taxonomy names" are bacteria which have DNA sharing 94% or more homology with the nucleotide sequence of 16S rRNA gene in bacteria found under the species names shown in Table 1A.

Blautia, Clostridium, unclassified Clostridiales, Actinomyces, Parabacteroides, Lachnoclostridium, Bacteroides, Faecalibacterium, unclassified Lachnospiraceae, Roseburia, Ruminococcus, unclassified Firmicutes, Dorea, Phascolarctobacterium, Sutterella, Megamonas, Collinsella, Eubacterium, Coprococcus, Schaalia, Alistipes, Bifidobacterium, Lactobacillus, Veillonella, Anaerostipes, Bilophila, Butyricicoccus, Barnesiella, Fusicatenibacter, Flavonifractor, unclassified Ruminococcaceae, unclassified Clostridiaceae, Faecalicatena, Prevotella, Megasphaera, Robinsoniella, Faecalitalea, Lachnospira, Romboutsia

Thus, the composition of the present invention comprises bacteria belonging to any genus selected from the group consisting of the following genera: Blautia, Clostridium, unclassified Clostridiales, Actinomyces, Parabacteroides, Lachnoclostridium, Bacteroides, Faecalibacterium, unclassified Lachnospiraceae, Roseburia, Ruminococcus, unclassified Firmicutes, Dorea, Phascolarctobacterium, Sutterella, Megamonas, Collinsella, Eubacterium, Coprococcus, Schaalia, Alistipes, Bifidobacterium, Lactobacillus, Veillonella, Anaerostipes, Bilophila, Butyricicoccus, Barnesiella, Fusicatenibacter, Flavonifractor, unclassified Ruminococcaceae, unclassified Clostridiaceae, Faecalicatena, Prevotella, Megasphaera, Robinsoniella, Faecalitalea, Lachnospira, and Romboutsia.

More preferably, the composition of the present invention comprises bacteria belonging to any genus selected from the group consisting of the following genera: Blautia, Clostridium, unclassified Clostridiales, Actinomyces, Parabacteroides, Lachnoclostridium, Bacteroides, Faecalibacterium, unclassified Lachnospiraceae, Roseburia, Ruminococcus, unclassified Firmicutes, Dorea, Phascolarctobacterium, Sutterella, Megamonas, Collinsella, Eubacterium, and Coprococcus.

### 3-3. Definition by species name

In the present invention, Method 1 (narrow down search with median) and Method 2 (narrow down search with mean and carrying rate) mentioned above can also be applied to define preferred bacterial species. For definition of species names, a narrow down search was made for bacterial species belonging to the genus names defined in the section "3-2. Definition by genus name."

In the present invention, bacteria determined to be preferred by Method 1 (narrow down search with median) are of the following species.

### [Table 1B]

**Table IB**

| Narrow down search with median |
|---|
| Bacteroides uniformis |
| Bacteroides sp. AR29 |
| Bacteroides ovatus |
| Bacteroides dorei |
| Bacteroides vulgatus |
| Parabacteroides distasonis |
| Bacteroides stercoris |
| Schaalia odontolytica |
| Parabacteroides merdae |
| Bifidobacterium longum |
| Bacteroides faecis |
| Dorea longicatena |
| Ruminococcus sp. K-1 |
| Blautia wexlerae |
| Blautia massiliensis |
| Bacteroides fragilis |
| [Clostridium] bolteae |
| Bifidobacterium adolescentis |
| [Ruminococcus] torques |
| butyrate-producing bacterium M104/1 |
| Bifidobacterium faecale |
| Anaerostipes hadrus |
| Clostridium sp. 826 |
| Collinsella aerofaciens |
| Clostridium sp. HGF2 |
| Clostridium sp. AT4 |
| Bilophila sp. 4_1_30 |
| Faecalibacterium prausnitzii |
| [Ruminococcus] gnavus |
| Roseburia inulinivorans |
| Bifidobacterium pseudocatenulatum |
| Butyricicoccus faecihominis |
| Firmicutes bacterium DJF_VR50 |
| Lachnospiraceae bacterium 1_1_57FAA |
| Blautia obeum |
| Ruminococcus sp. DJF_VR70k1 |

Likewise, in the present invention, bacteria determined to be preferred by Method 2 (narrow down search with mean and carrying rate) are of the following species.

### [Table 1C]

**Table 1C**

| Narrow down search with mean and carrying rate | |
|---|---|
| Bacteroides vulgatus | Eubacterium ventriosum |
| Bacteroides stercoris | Clostridiales bacterium 80/3 |
| Bacteroides uniformis | Butyricicoccus faecihominis |
| Blautia wexlerae | Bacteroides cellulosilyticus |
| Bacteroides sp. AR29 | Coprococcus catus |
| Dorea longicatena | Parabacteroides johnsonii |
| Phascolarctobacterium faecium | Lachnospiraceae bacterium DJF_RP14 |
| Blautia massiliensis | [Ruminococcus] gnavus |
| Ruminococcus sp. K-1 | Bifidobacterium longum |
| Faecalibacterium prausnitzii | Bacteroides xylanisolvens |
| Megamonas funiformis | Prevotella stercorea |
| Bifidobacterium adolescentis | Bacteroides plebeius |
| [Ruminococcus] torques | Firmicutes bacterium DJF_VR50 |
| Collinsella aerofaciens | Sutterella wadsworthensis |
| Parabacteroides merdae | Lachnospiraceae bacterium 1_1_57FAA |
| butyrate-producing bacterium M104/1 | Blautia obeum |
| Bifidobacterium faecale | Barnesiella intestinihominis |
| Clostridium sp. 826 | [Eubacterium] eligens |
| Bacteroides coprocola | Coprococcus comes |
| Roseburia faecis | Ruminococcus sp. DJF_VR70k1 |
| Lachnospiraceae bacterium DJF_VP18k1 | butyrate-producing bacterium A2-175 |
| Clostridium sp. AT4 | Faecalitalea cylindroides |
| Lactobacillus rogosae | Blautia sp. YHC-4 |
| butyrate-producing bacterium A2-207 | [Clostridium] glycyrrhizinilyticum |
| Roseburia sp. 1120 | Ruminococcus sp. 653 |
| Bacteroides dorei | Ruminococcus lactaris |
| Bifidobacterium pseudocatenulatum | butyrate-producing bacterium SM6/1 |
| Megasphaera massiliensis | butyrate-producing bacterium SS3/4 |
| Bacteroides massiliensis | Clostridiaceae bacterium DJF_LS40 |
| Anaerostipes hadrus | Bacteroides sp. 1_1_30 |
| Alistipes putredinis | Bacteroides nordii |
| Parabacteroides sp. D25 | Fusicatenibacter saccharivorans |
| Roseburia inulinivorans | butyrate-producing bacterium SL7/1 |
| Bilophila sp. 4_1_30 | [Clostridium] scindens |
| Flavonifractor plautii | |

Species extracted by the above two methods for narrow down search are summarized as follows.

### [Table ID]

**Table ID**

| Species | Species |
|---|---|
| Blautia wexlerae | Roseburia faecis |
| Blautia massiliensis | Roseburia sp. 1120 |
| [Ruminococcus] torques | Roseburia inulinivorans |
| [Ruminococcus] gnavus | Ruminococcus sp. K-1 |
| Blautia obeum | Ruminococcus sp. DJF_VR70k1 |
| Blautia sp. YHC-4 | Ruminococcus sp. 653 |
| Clostridium sp. 826 | Ruminococcus lactaris |
| Clostridium sp. AT4 | Firmicutes bacterium DJF_VR50 |
| Clostridium sp. HGF2 | Dorea longicatena |
| butyrate-producing bacterium M104/1 | Phascolarctobacterium faecium |
| butyrate-producing bacterium A2-207 | Sutterella wadsworthensis |
| Clostridiales bacterium 80/3 | Megamonas funiformis |
| butyrate-producing bacterium A2-175 | Collinsella aerofaciens |
| butyrate-producing bacterium SM6/1 | Eubacterium ventriosum |
| butyrate-producing bacterium SS3/4 | [Eubacterium] eligens |
| butyrate-producing bacterium SL7/1 | Coprococcus catus |
| | Coprococcus comes |
| Parabacteroides merdae | Schaalia odontolytica |
| Parabacteroides sp. D25 | Alistipes putredinis |
| Parabacteroides johnsonii | Bifidobacterium adolescentis |
| Parabacteroides distasonis | Bifidobacterium faecale |
| [Clostridium] glycyrrhizinilyticum | Bifidobacterium pseudocatenulatum |
| [Clostridium] scindens | Bifidobacterium longum |
| [Clostridium] bolteae | Lactobacillus rogosae |
| Bacteroides vulgatus | |
| Bacteroides stercoris | Anaerostipes hadrus |
| Bacteroides uniformis | Bilophila sp. 4_1_30 |
| Bacteroides sp. AR29 | Butyricicoccus faecihominis |
| Bacteroides ovatus | Barnesiella intestinihominis |
| Bacteroides coprocola | Fusicatenibacter saccharivorans |
| Bacteroides dorei | Flavonifractor plautii |
| Bacteroides massiliensis | |
| Bacteroides cellulosilyticus | Clostridiaceae bacterium DJF_LS40 |
| Bacteroides xylanisolvens | |
| Bacteroides plebeius | Prevotella stercorea |
| Bacteroides sp. 1_1_30 | Megasphaera massiliensis |
| Bacteroides nordii | |
| Bacteroides faecis | Faecalitalea cylindroides |
| Bacteroides fragilis | |
| Faecalibacterium prausnitzii | |
| Lachnospiraceae bacterium DJF_VP18k1 | |
| Lachnospiraceae bacterium DJF_RP14 | |
| Lachnospiraceae bacterium 1_1_57FAA | |

In the present invention, the results of the narrow down search for species indicate that 76 species shown in Table 1D (referred to as Group A) are abundant in CR or PR. Among them, the following 27 species (referred to as Group B) are more preferred. Among these species falling within Group B, the following 10 species (referred to as Group C) are most preferred.

### Group B (27 species):

Bacteroides vulgatus
Bacteroides stercoris
Bacteroides uniformis
Blautia wexlerae
Bacteroides sp. AR29
Dorea longicatena
Phascolarctobacterium faecium
Blautia massiliensis
Ruminococcus sp. K-1
Bacteroides ovatus
Faecalibacterium prausnitzii
Megamonas funiformis
Bifidobacterium adolescentis
[Ruminococcus] torques
Collinsella aerofaciens
Parabacteroides merdae
butyrate-producing bacterium M104/1
Bifidobacterium faecale
Clostridium sp. 826
Bacteroides coprocola
Roseburia faecis
Lachnospiraceae bacterium DJF_VP18k1
Clostridium sp. AT4
Lactobacillus rogosae
butyrate-producing bacterium A2-207
Roseburia sp. 1120
Bacteroides dorei

### Group C:

Bacteroides vulgatus
Bacteroides stercoris
Bacteroides uniformis
Blautia wexlerae
Bacteroides sp. AR29
Dorea longicatena
Phascolarctobacterium faecium
Blautia massiliensis
Ruminococcus sp. K-1
Bacteroides ovatus

In the composition of the present invention, bacteria belonging to the genera listed above may be contained alone, or some of these bacteria may be contained in combination. When some of these bacteria are contained in combination, up to 40 types of bacteria are preferred, up to 30 type of bacteria are more preferred, and up to 20 types of bacteria are most preferred. It is possible to select any combination of up to 20 types of bacteria, for example, any combination of 20, 19, 18, 17, 16, 15, 14, 13, 12, 11, 10, 9, 8, 7, 6, 5, 4, 3 or 2 types of bacteria.

Moreover, the composition of the present invention may be in any powder, solid or liquid form in order that it is used as appropriate in allo-HSCT or can be used for a long period of time. Such a powder, solid or liquid form may also be formulated into a capsule formulation. The composition of the present invention when formulated into a capsule formulation is advantageous in that it is possible to avoid complications such as hemorrhage caused by tube insertion and/or a colon fiberscope, etc., and it is also possible to reduce the burdens on patients during implementation.

Further, the composition of the present invention may comprise either live bacteria or dead bacteria, or may comprise a mixture of live and dead bacteria.

Furthermore, the composition of the present invention may be configured to comprise at least one selected from pH stabilizers, acidifiers, antiseptics, vitamins, minerals, nutritional supplements, prebiotics and probiotics.

Moreover, the composition of the present invention may be administered either before or after or both before and after hematopoietic stem cell transplantation to a patient who is a subject of this transplantation, whereby GVHD can be prevented or treated.

GVHD may be exemplified by steroid-refractory or steroid-dependent GVHD, or acute gut GVHD

In the context of the term "treatment" or "therapeutic" as used herein, the degree of suppression is not limited in any way as long as the development of GVHD can be suppressed. Thus, the "treatment" includes both complete response (CR) and partial response (PR). Complete response (CR) means the disappearance of all gut GVHD-related symptoms, while partial response (PR) means at least one down staging of gut GVHD. In the present invention, the treatment is assumed to be effective (i.e., GVHD has been treated) when reaching PR or CR in steroid-resistant cases or when succeeding in 40% or more steroid reduction in steroid-dependent cases as compared to before treatment.

Likewise, the term "prevention" or "prophylactic" is intended to include all of the following meanings: to suppress the development of GVHD before it occurs, to prevent the condition of already developed GVHD from becoming worse, and to prevent the recurrence of resolved GVHD

In the present invention, there is provided a therapeutic method for GVHD, which comprises administering the above composition or capsule formulation to a GVHD patient.

The present invention further provides a prophylactic method for GVHD, which comprises administering the above composition or capsule formulation either before or after or both before and after hematopoietic stem cell transplantation to a patient who is a subject of the hematopoietic stem cell transplantation.

### 4. Bacterial mixture and formulation

The present invention provides a method for preparing a suspension for fecal microbiota transplantation, which comprises confirming the presence of the above bacteria in feces collected from a human subject, and suspending the feces confirmed for the presence of the bacteria into a solvent (e.g., physiological saline, buffer).

Moreover, the present invention provides a method for preparing a suspension for fecal microbiota transplantation, which comprises confirming the presence of the above bacteria in feces collected from a human subject, obtaining a microbiota from the feces confirmed for the presence of the bacteria, and suspending the microbiota into a solvent.

Further, the present invention provides a method for preparing a bacterial mixture for fecal microbiota transplantation, which comprises confirming the presence of the above bacteria in feces collected from a human subject, separating the bacteria from the feces confirmed for the presence of the bacteria, and mixing the separated bacteria. In the separation of bacteria, bacteria contained in a sample may be of a single type or may be of two or more types.

In yet another embodiment of the present invention, there is provided a method for preparing a formulation for fecal microbiota transplantation, which comprises confirming the presence of the above bacteria in feces collected from a human subject, and formulating a microbiota from the feces confirmed for the presence of the bacteria, or the bacteria separated from the feces confirmed for the presence of the bacteria.

Formulation techniques are exemplified by encapsulation into capsules.

Feces may be collected according to the procedures described in the section "1. FMT" above.

Techniques used to confirm the presence of bacteria are not limited in any way, and examples include 16S rRNA gene analysis, qPCR or Microarray using the DNA sequences of regions specific to bacteria of interest, or MALDI-TOF MS, etc.

Once the presence of bacteria has been confirmed by 16S rRNA gene analysis or other techniques, it can be assumed that the bacteria are contained in the donor feces. A diluted suspension of such feces is subjected to various culture conditions, and single colonies are collected to isolate the bacteria.

The formulation of the present invention may be in any dosage form as long as it comprises the above bacteria of the present invention, but preferred is a formulation for oral administration.

In the case of preparing an oral solid formulation, a base drug may be supplemented with an excipient and optionally with a binder, a disintegrant, a lubricant, a coloring agent, a corrective and so on, and then formulated in a standard manner into tablets, coated tablets, granules, fine granules, powders, capsules, etc.

Capsules used for this purpose include, for example, acid-resistant capsules. Such acid-resistant capsules may be exemplified by DR Caps^{®} (Capsugel).

Examples of an excipient available for use include lactose, corn starch, sucrose, glucose, sorbit, crystalline cellulose, silicon dioxide and so on. Examples of a binder available for use include polyvinyl alcohol, ethyl cellulose, methyl cellulose, gum arabic, hydroxypropyl cellulose, hydroxypropyl methyl cellulose and so on. Examples of a lubricant available for use include magnesium stearate, talc, silica and so on. Examples of a coloring agent available for use include those permitted to be added to medicaments. Examples of a corrective available for use include cocoa powder, menthol, aromatic acids, peppermint oil, borneol, cinnamon powder and so on.

In the present invention, a capsule formulation is preferred among the above formulations.

Bacteria contained in the formulation may be set to, for example, 1 to 2000 mg, preferably 10 to 500 mg, and more preferably 100 to 250 mg by dry weight, and may be administered in an amount of 150 to 200 mg per day, preferably 175 mg per day, depending on the patient's body weight and symptoms, etc.

The bacteria intended in the present invention may be used either as a medicament or as a quasi drug.

### EXAMPLES

The present invention will be further described in more detail by way of the following illustrative examples. However, the scope of the present invention is not limited by these examples.

### Example 1

FMT for screening of preferred microbiota

### [Subjects and Methods]

### Subjects of FMT

FMT was performed on 15 cases of steroid-refractory or steroid-dependent acute gut GVHD.

Not only the gut, but also the skin and the liver can be target organs of acute GVHD, but this study was targeted at the gut in terms of the fact that acute gut GVHD is more severe than acute GVHD in the other organs. A steroid-refractory case refers to a case where in spite of treatment with adequate steroid dose (1 mg/kg or more of prednisolone), the clinical condition of a patient remains unchanged from day 5 after initiation of the treatment, while a steroid-dependent case refers to a case where a patient initially responds to steroid treatment, but becomes worse with reduction in steroid dose, so that the steroid dose is required to be increased again (i.e., a case where steroid dose is difficult to reduce).

In addition to the above cases, this study was also targeted at cases where acute gut GVHD progressed on day 3 after initiation of steroid treatment.

Even in the case of patients complicated with other gut lesions, cases where acute gut GVHD was considered to be the leading cause of diarrhea were available for entry.

Exclusion criteria were set as shown below.
1) GVHD responsive to steroid treatment
2) GVHD progressive after primary steroid treatment (regardless of affected organ)
3) Cases with uncontrollable infections
4) Cases where diarrhea is deemed to result from any cause other than GVHD

### Selection of FMT donors

Donor candidates were selected from the spouses or relatives within the fourth degree of relationship of patients. These candidates were between 20 and 64 years of age and were selected from those who did not have any infection risks as shown below.
1) Not have gotten a new tattoo or pierced within the last three months
2) Not have had sexual contact with a new partner within the last three months
3) Not have received a blood transfusion within the last three months
4) Not have had a travel history to tropical regions within the last three months
5) Not have used any antibiotics within the last one month
6) Not have a previous history of malignant disease or inflammatory bowel disease
7) Not have any digestive symptoms such as diarrhea on the day of FMT (as confirmed on the day of FMT)
8) Not have hypertension, diabetes, hyperlipidemia, cardiac diseases (e.g., myocardial infarction, angina pectoris, heart failure), apoplexy, or allergic diseases (e.g., asthma, atopic dermatitis, food allergy) (except for pollinosis)

If donor candidates have no problem with the above items, they were subjected to blood collection and fecal examination. Blood examination was made to check HIV, human T-lymphotropic virus type I (HTLV-1), hepatitis A, B and C, syphilis, cytomegalovirus (CMV) and Epstein-Barr virus (EBV). Likewise, fecal examination was made to check the presence or absence of parasites, Clostridium difficile, Cryptosporidium, Giardia, Microsporidia, Entamoeba histolytica, Cyclospora, Isospora, Dientamoeba fragilis, Blastocystis hominis, Schistosoma and other pathogenic bacteria, etc. With regard to CMV and EBV, patients showing a past infection pattern of CMV or EBV were determined to have no problem.

### FMT method

On the day of FMT, feces were collected from the donors and stored at 4°C under anaerobic conditions until use.

At the timing of inserting a gastroduodenal tube into each patient, feces were started to be prepared. For preparation of feces, feces were first weighed, and sterile physiological saline was added in a volume of 200 to 300 mL depending on the weight of feces, followed by thoroughly stirring to give a uniform mixture. This mixture was passed once through a metal sieve to remove large undigested matters, and then passed twice through sterile gauze to prepared a suspension. FMT was performed as soon as patients were ready.

The suspension was filled into 50 mL syringes, and the fecal suspension was administered through the gastroduodenal tube. The administration rate was set not to exceed 30 seconds per 50 mL. After the entire suspension was introduced, 50 mL of physiological saline was used to wash the inside of the tube, and the tube was then removed to complete the administration.

When no FMT-related adverse event of grade 3 (as defined in Non-patent Document 3) or higher was observed, additional administration was allowed once within 4 to 14 days after the first FMT while checking its effect. In this case, feces were collected from the same donor as used for the first administration. Fecal transplantation was performed within 12 hours (within 8 hours, if possible) after fecal collection from the donor.

Patients who were fasting due to their gut GVHD were allowed to ingest supplements containing dietary fiber, oligosaccharides and so on (GFO^{®}: Otsuka Pharmaceutical Co., Ltd., Japan) as prebiotics from the day before initiation of FMT. Moreover, the administration of antibiotics was stopped for 24 hours before and after FMT, if possible.

Cases newly developed or showing progression of grade 1 (as defined in Non-patent Document 3) or higher within one week after FMT were all regarded as adverse events, and evaluated by the National Cancer Institute Common Terminology Criteria for Adverse Events (CTC-AE) version 4.0.

The therapeutic effect was evaluated at 4 weeks after the final FMT. Criteria for therapeutic effect assessment are as shown below.
1) Complete response (CR): disappearance of all gut GVHD-related symptoms
2) Partial response (PR): at least one down staging of gut GVHD
3) Progression (PG): progression of at least one stage (as defined in Non-patent Document 3)
4) No change (NC): variation within the same stage (as defined in Non-patent Document 3)
5) Not determined (ND): early death (within 7 days) after treatment or any case difficult to evaluate due to symptoms other than GVHD

FMT was assessed to be effective when reaching PR or CR in steroid-resistant cases or when succeeding in 40% or more steroid reduction in steroid-dependent cases as compared to before treatment.

### Analysis of gut microbiota (meta 16S analysis)

Aliquots of the prepared fecal preparation and the patient's feces (collected before FMT, and at 1 to 3 days and 1, 2 and 4 weeks after the final FMT) were used for analysis of gut microbiota.

Bacterial DNA was extracted from each sample in a standard manner. This DNA was amplified by PCR (polymerase chain reaction) with primers (27Fmod: 5'-agrgtttgatymtggctcag-3' (SEQ ID NO: 345), 338R: 5'-tgctgcctcccgtaggagt-3' (SEQ ID NO: 346)) designed to cover the variable region v1-v2 in the 16S rRNA gene, followed by 250 bp paired-end sequencing on MiSeq^{®} (illumina). After Read 1 and Read 2 of the resulting sequence were merged, quality checking was conducted and 3,000 sequence data reads which passed this quality checking were used for analysis. The resulting reads were sorted in descending order by the abundance of the same sequence, and reads at the same abundance were sorted in descending order by the average of quality. The sorted sequences were subjected to operational taxonomic units (OTUs) clustering using the UCLUST algorithm with a homology threshold of 97%. Moreover, glsearch was used to perform homology search against 16S rRNA gene databases (RefSeq, RDP, GRD, CORE) to thereby identify bacterial species. The homology threshold in genus identification was set to 94%. The homology threshold in species identification was set to 97%.

Moreover, the Shannon index was used for α diversity evaluation. It should be noted that taxonomy names in this analysis were in accordance with taxdmp.zip on the FTP site of the NCBI Taxonomy Database as of May 7, 2019.

### Intergroup comparison of gut microbiota

The CR or PR group and the Others group were subjected to the two methods described in the sections "Method 1. Narrow down search with median" and "Method 2. Narrow down search for bacteria with mean and carrying rate" to extract enterobacteria abundant in the CR or PR group.

### [Results and Discussion]

In the therapeutic effect assessment using the results obtained at 4 weeks after the final administration, 6 of the 15 cases were assessed as CR and 3 of the 15 cases were assessed as PR, thus resulting in a response rate of 60%. Thus, this example demonstrated the effectiveness of FMT as a therapeutic or prophylactic strategy for GVHD

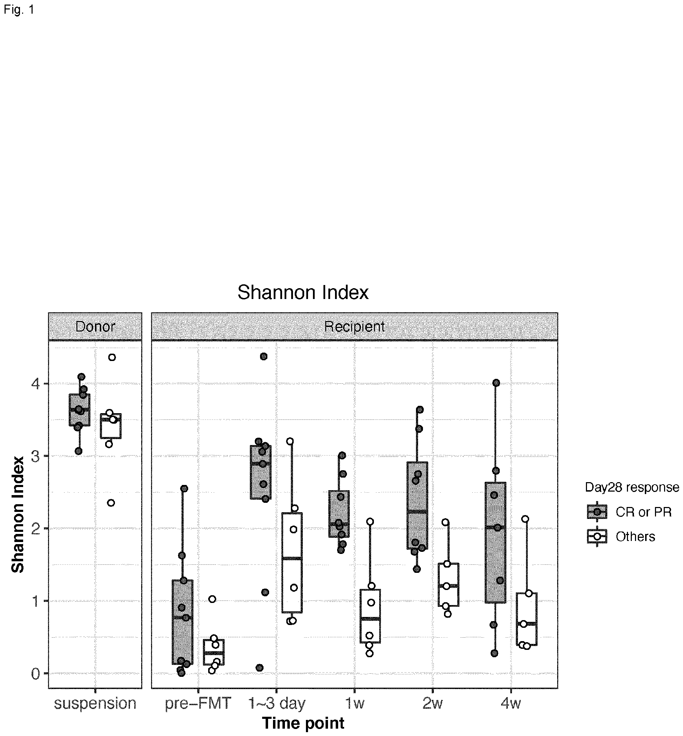

The time course of changes in the diversity of fecal microbiota before and after FMT is as shown in Figure 1. The CR or PR group and the Others group both showed an improvement in α diversity after FMT. The α diversity was gradually reduced over 4 weeks after FMT, but this reduction was suppressed in the CR or PR group as compared to the Others group.

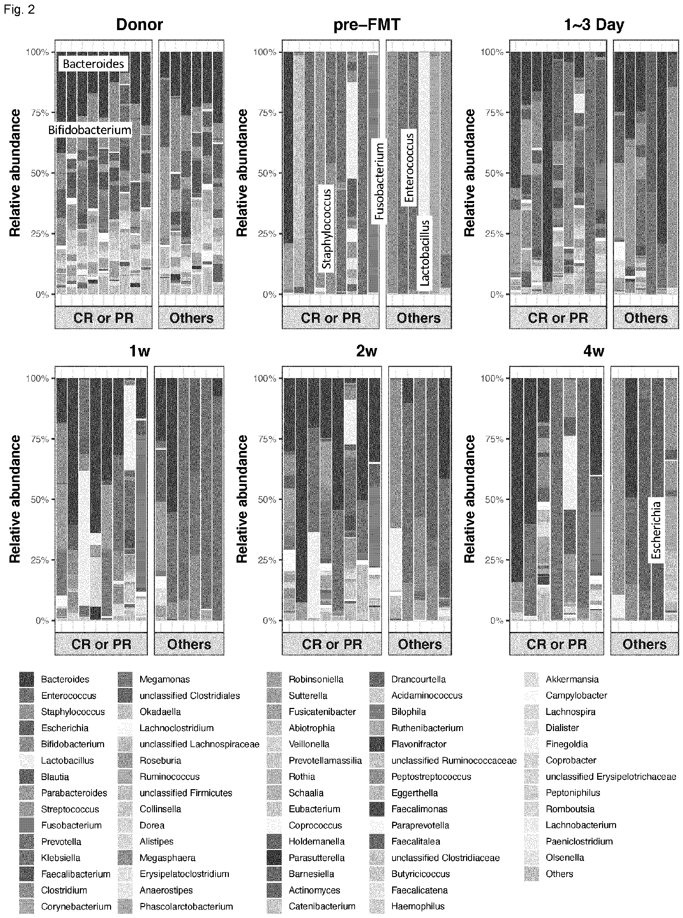

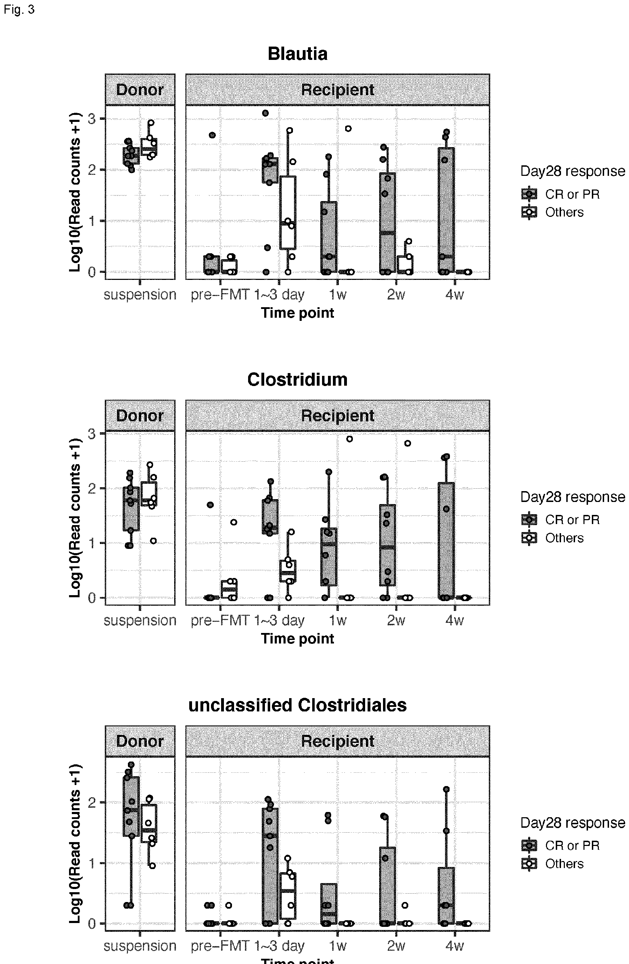

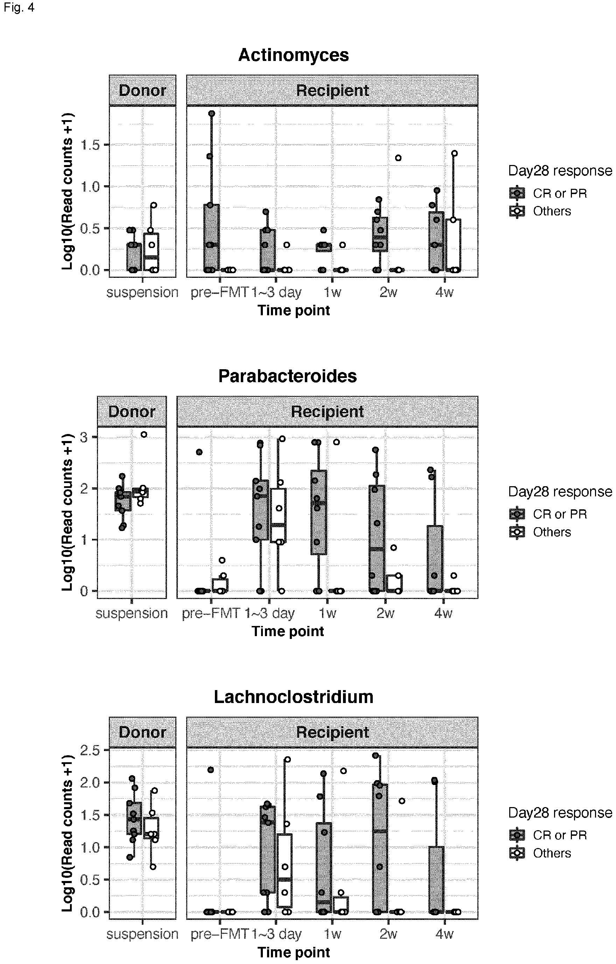

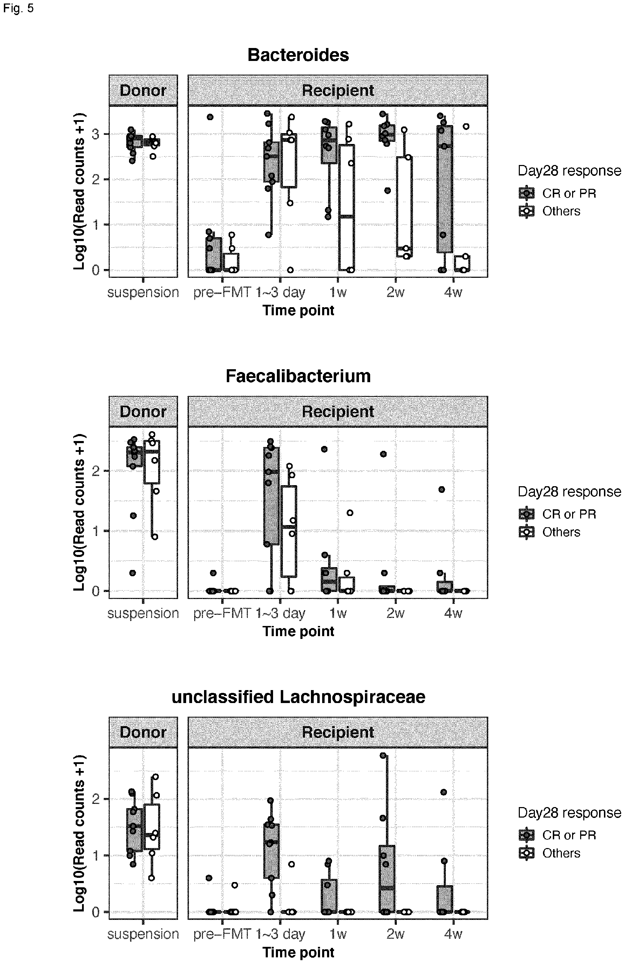

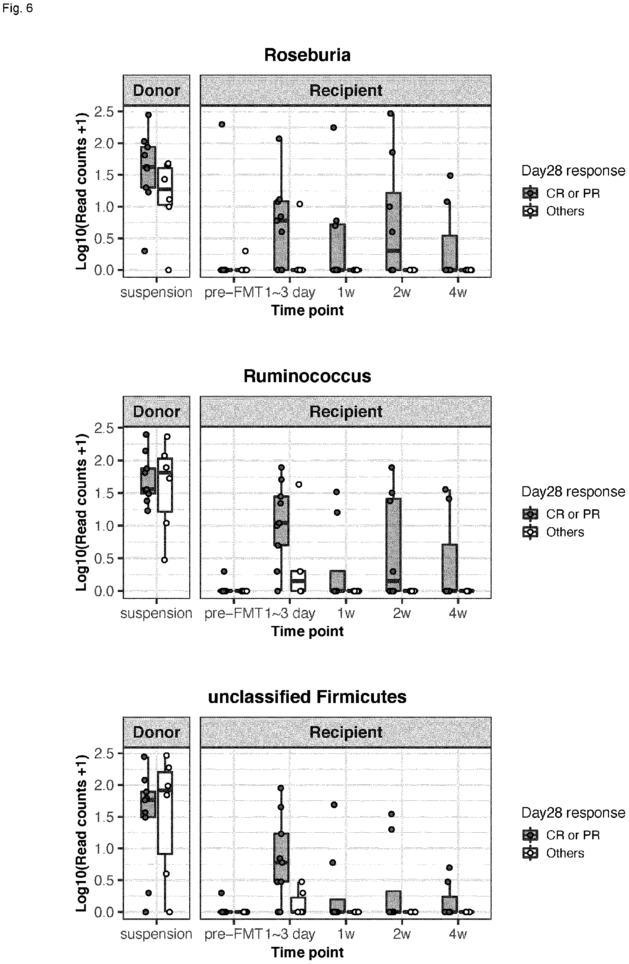

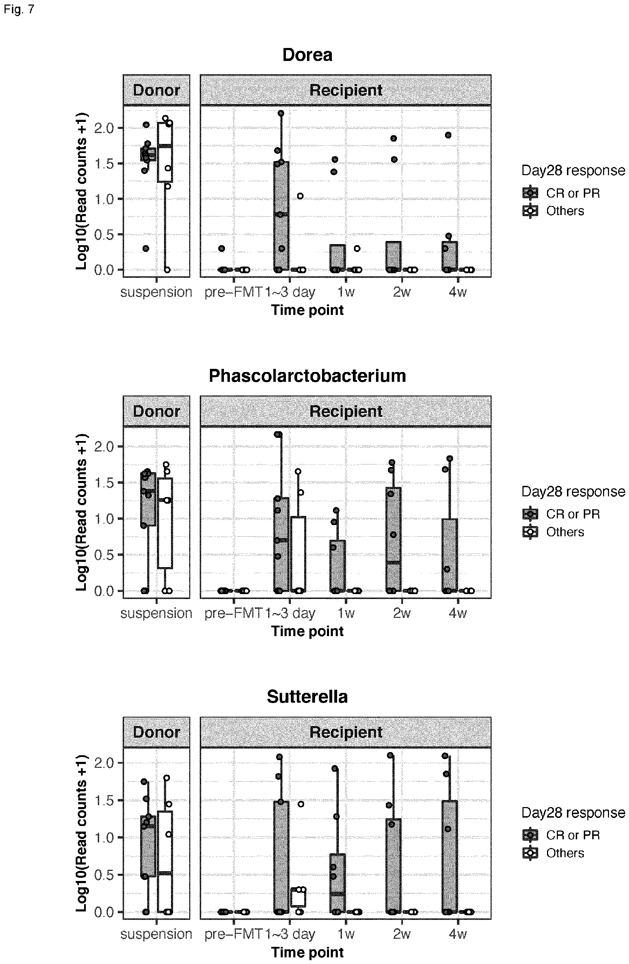

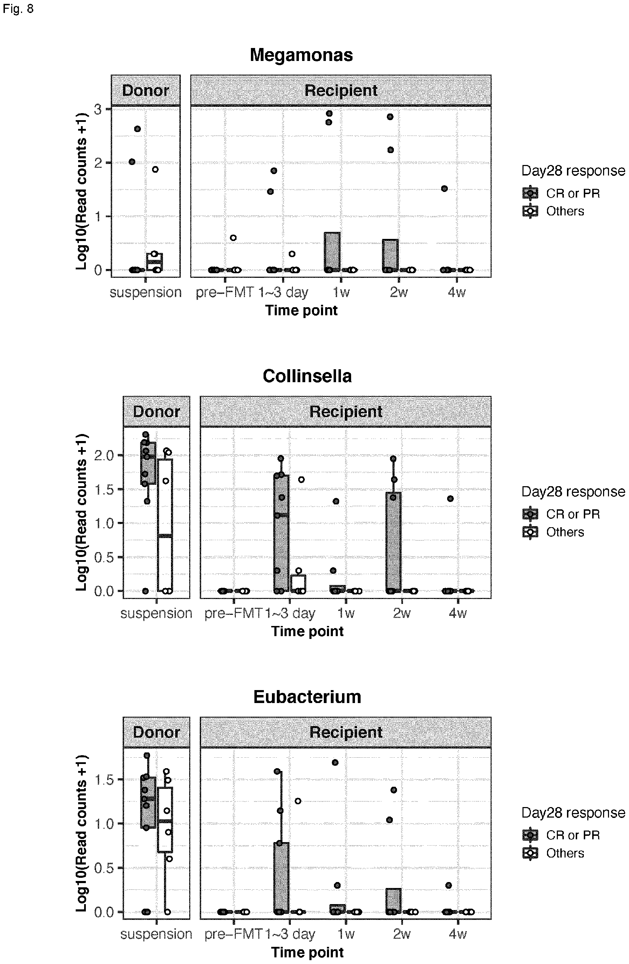

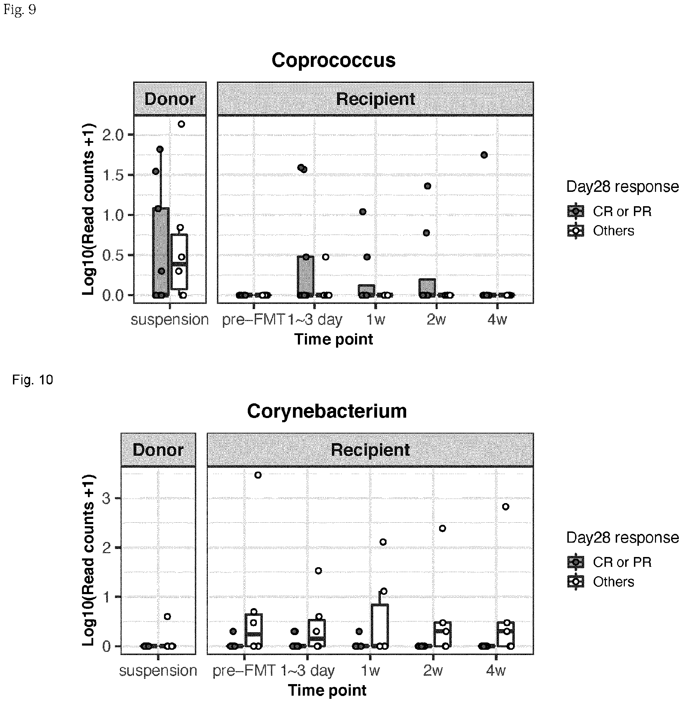

Graphs representing the relative proportions of fecal microbiota at the genus level before and after FMT are shown in Figure 2. Before FMT, the diversity of the microbiota was reduced (i.e., dysbiosis), and bacteria such as Staphylococcus and Enterococcus were dominant. After FMT, the diversity of the microbiota was recovered, so that the microbiota was altered to include various bacteria such as Bacteroides and Bifidobacterium. In the Others group, cases where Escherichia was dominant after 4 weeks were also observed. Moreover, for the respective genera, the time courses of their changes are shown in Figures 3 to 9. The genera shown in Figures 3 to 9 were all confirmed to be abundant in the CR or PR group in comparison with the Others group. Moreover, Corynebacterium shown in Figure 10 was confirmed to be abundant in the Others group at all the time points.

The results of two group comparison obtained by the two methods described in the sections "Method 1. Narrow down search with median" and "Method 2. Narrow down search for bacteria with mean and carrying rate" are shown below. The results at the OTU level are shown in Table 2 and Table 3, while the results at the genus level are shown in Table 4 and Table 5. These tables represent bacteria showing at least one time when the Log2 Fold change was 0.5 or more.

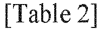

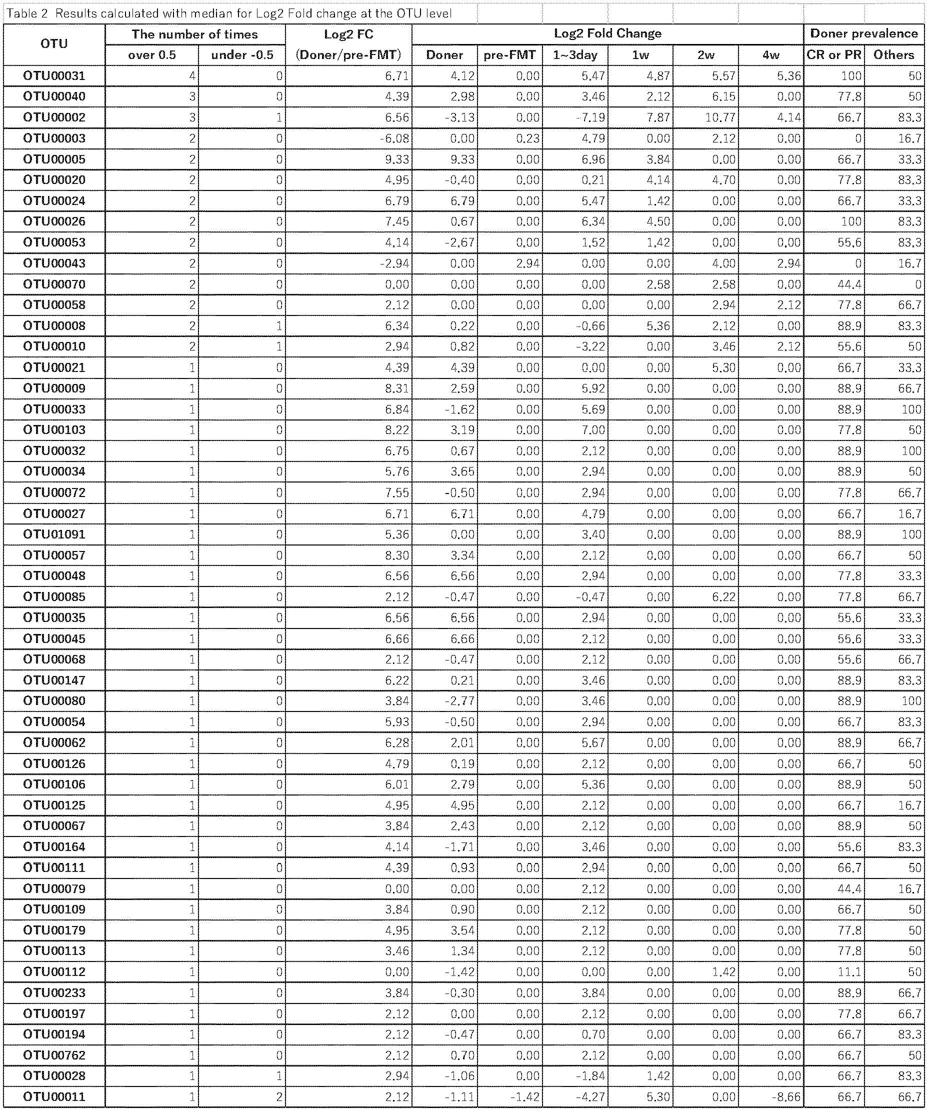

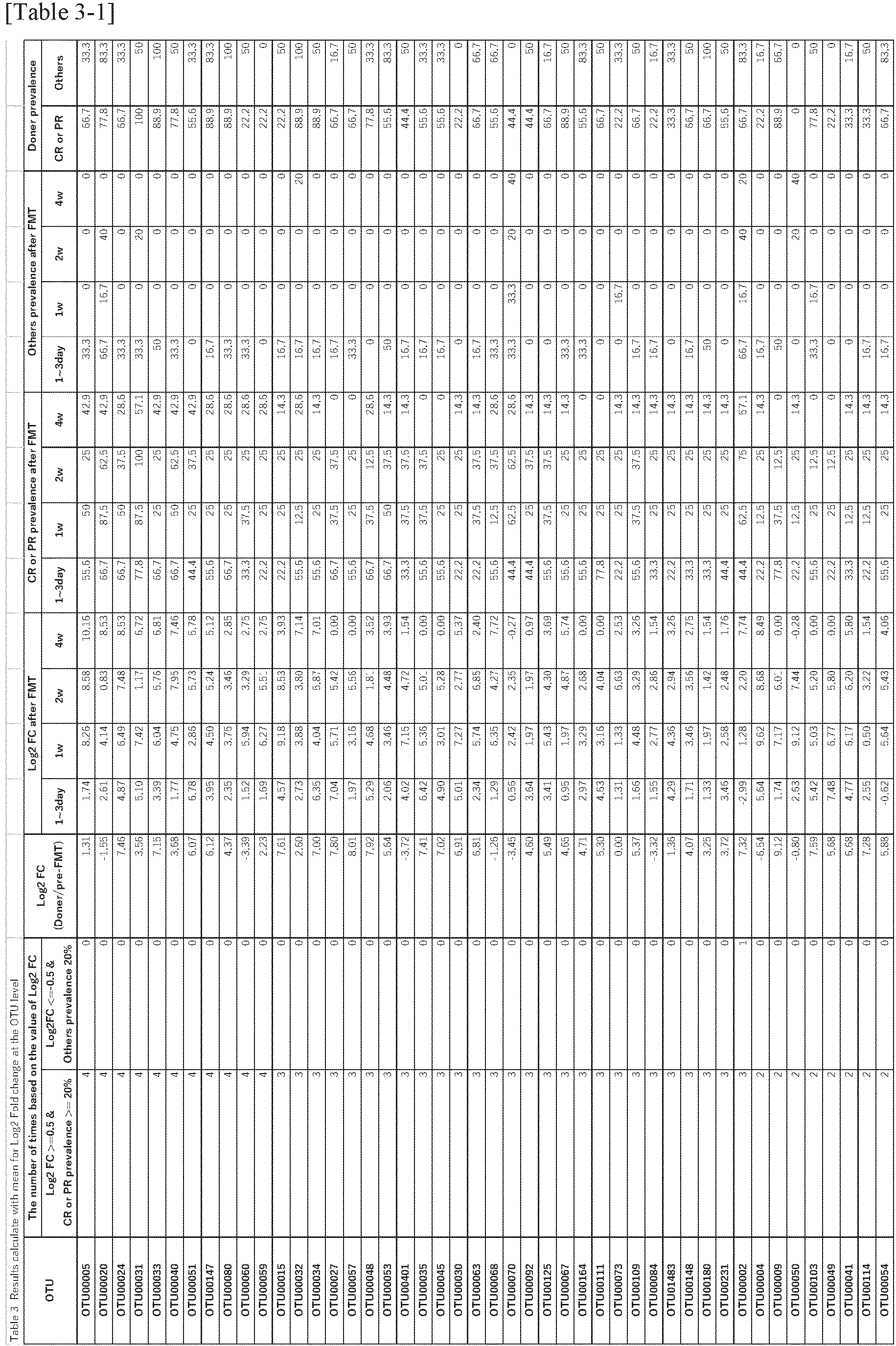

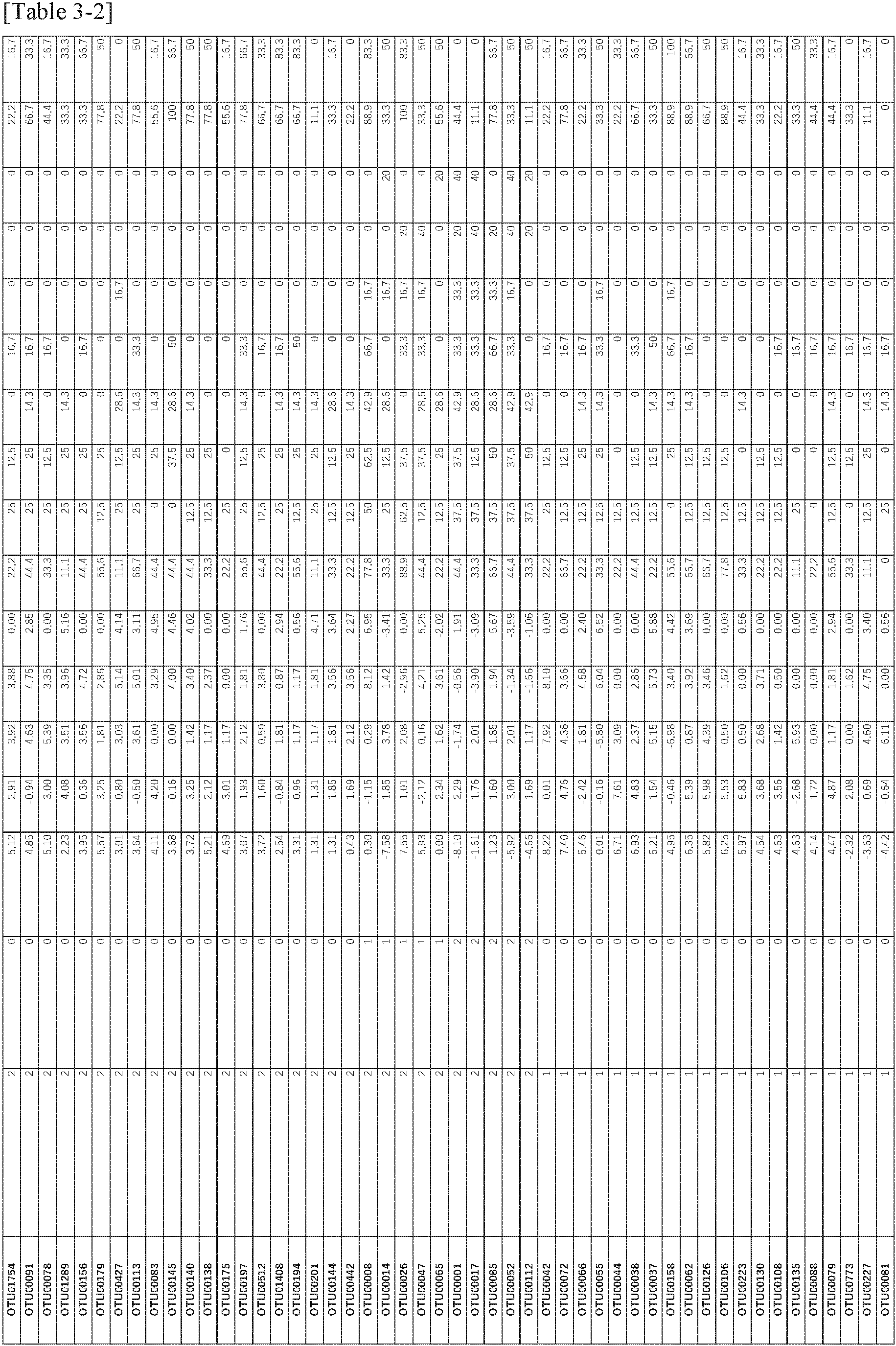

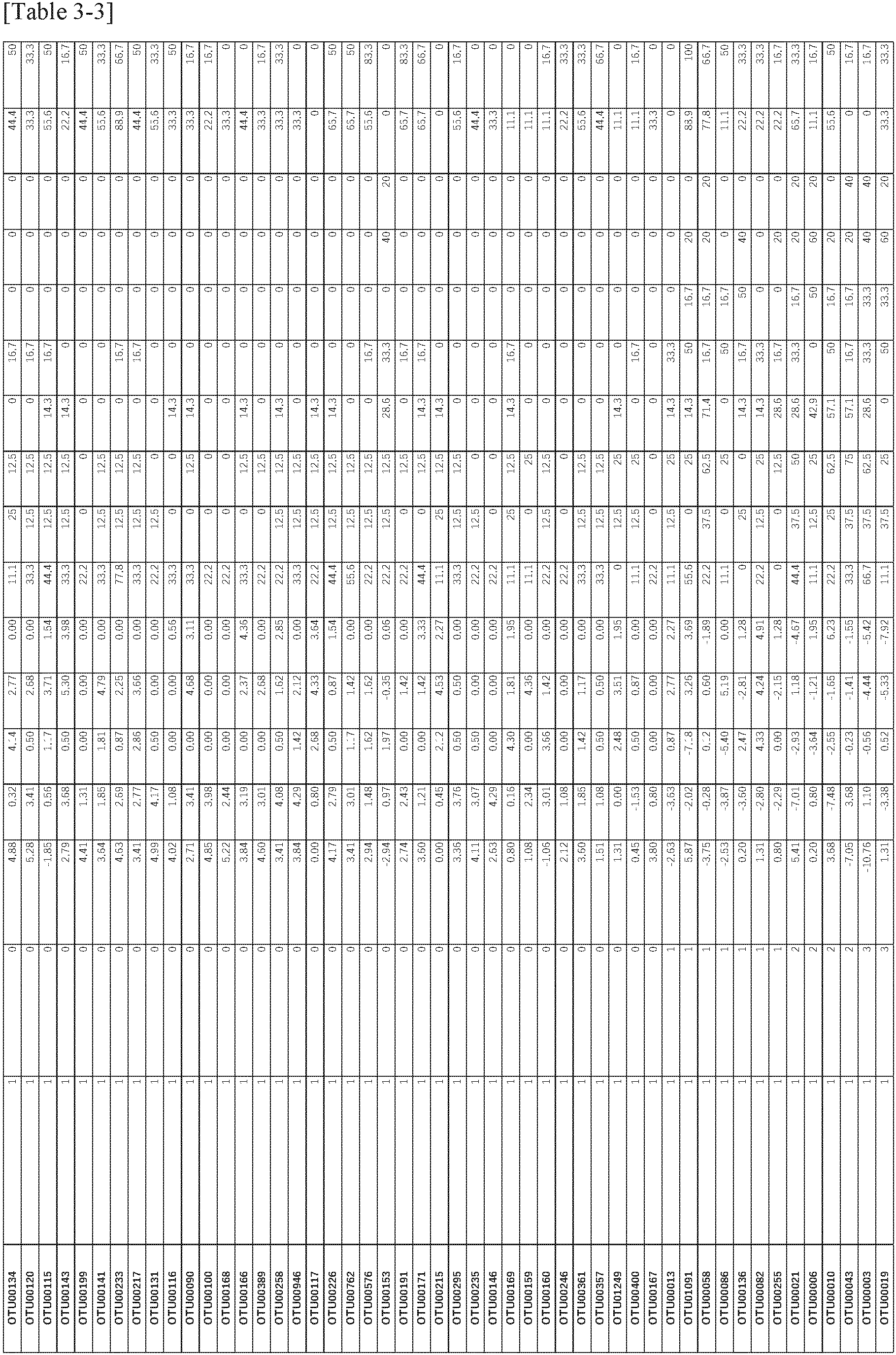

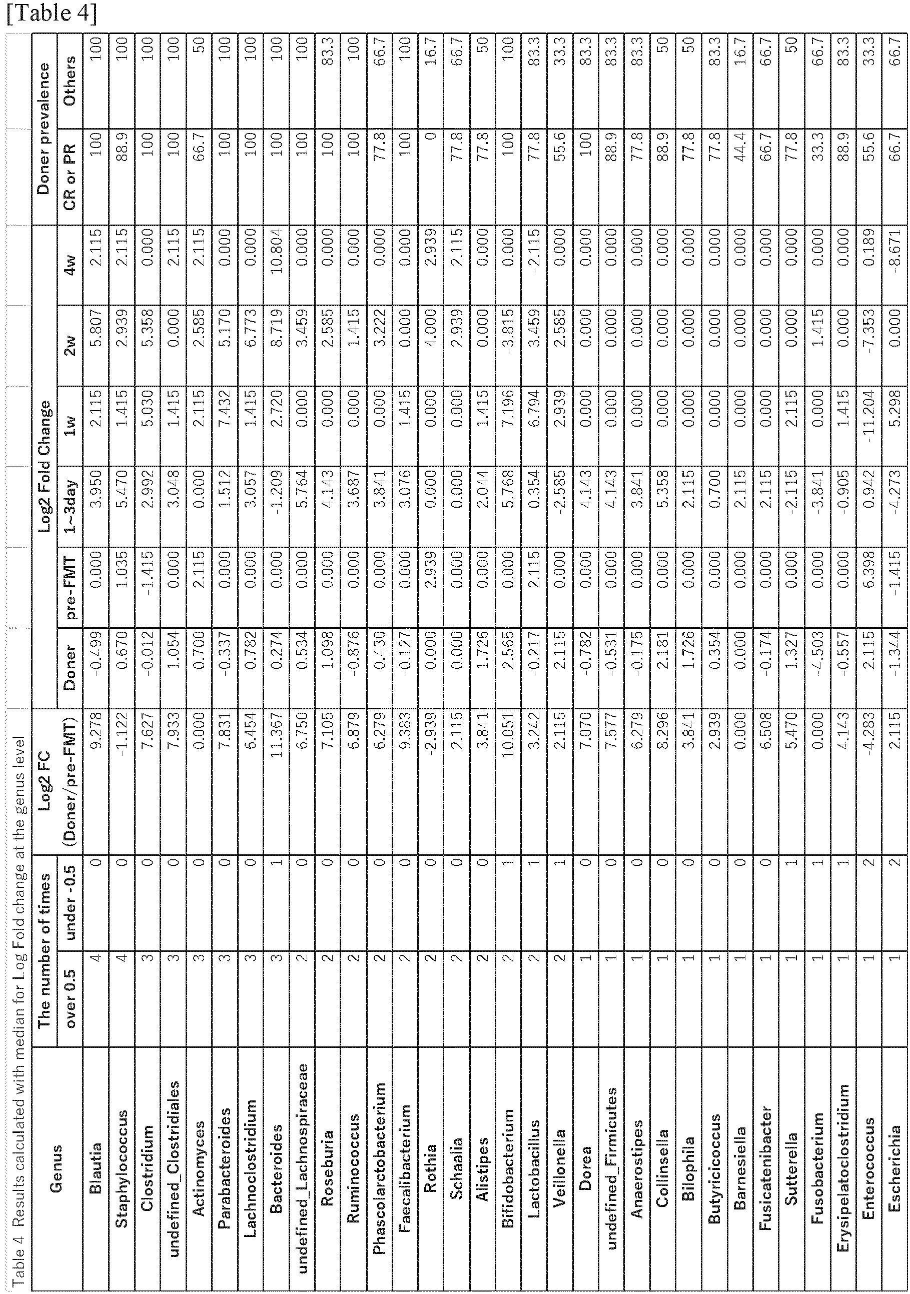

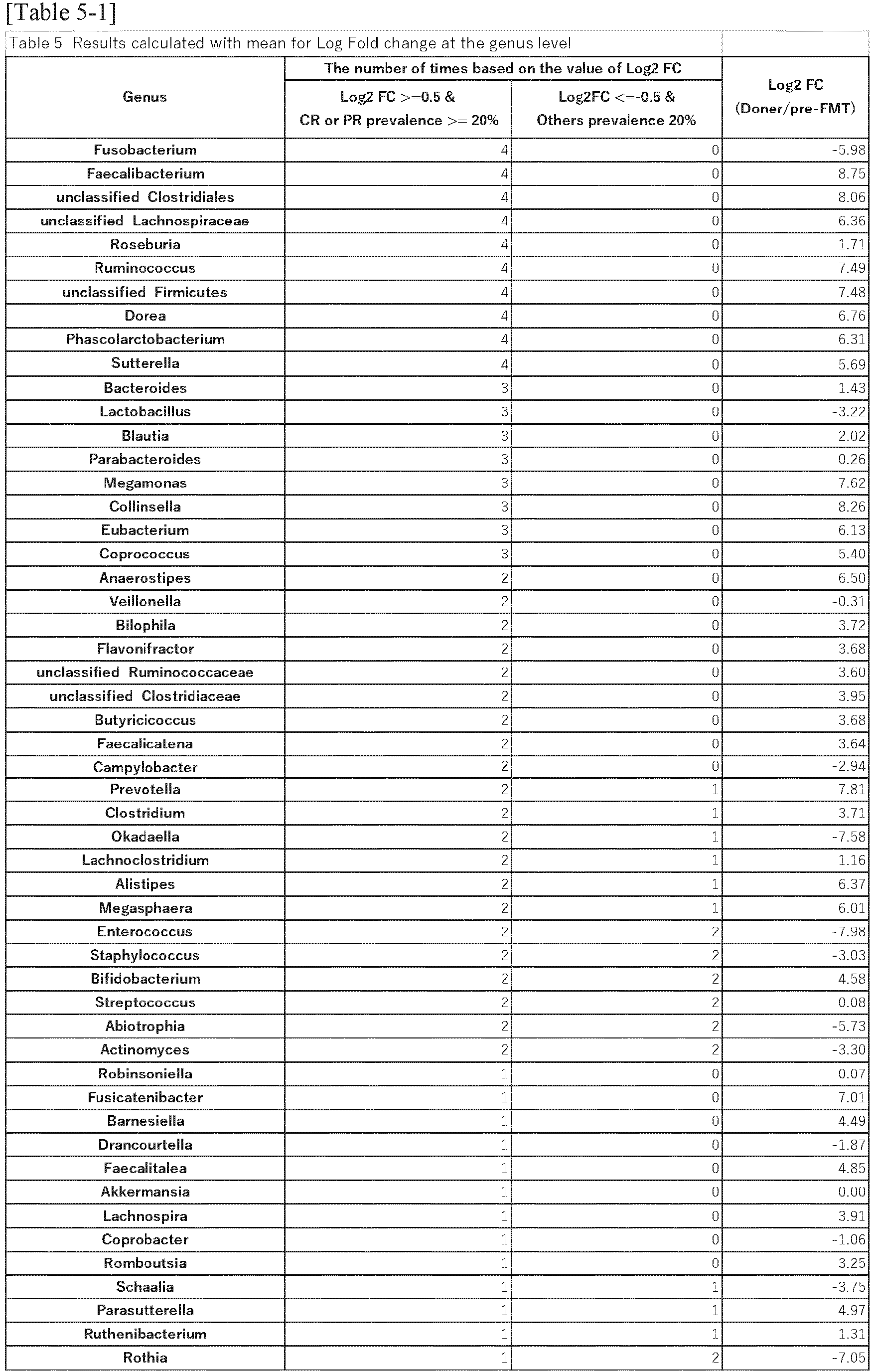

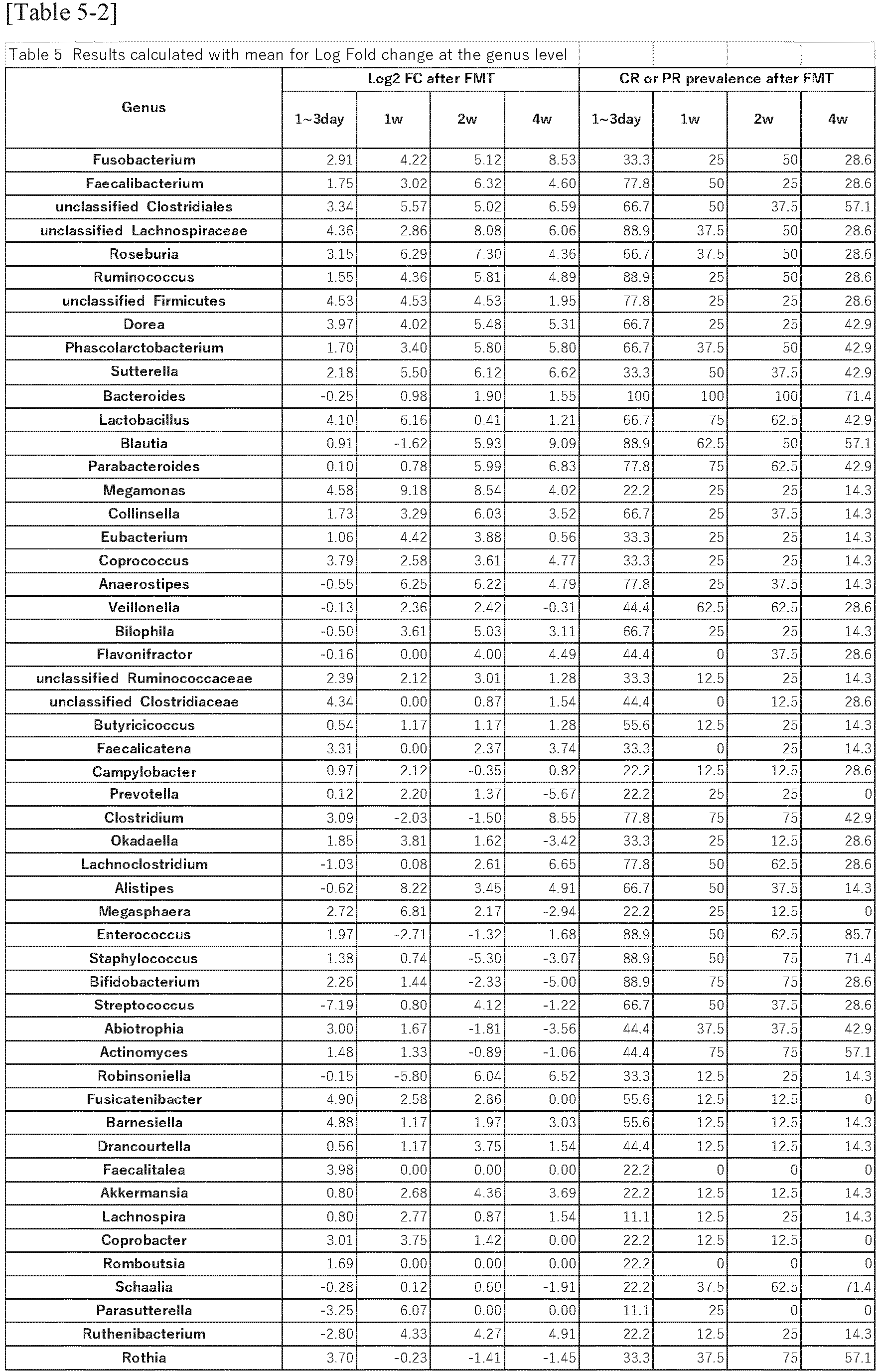

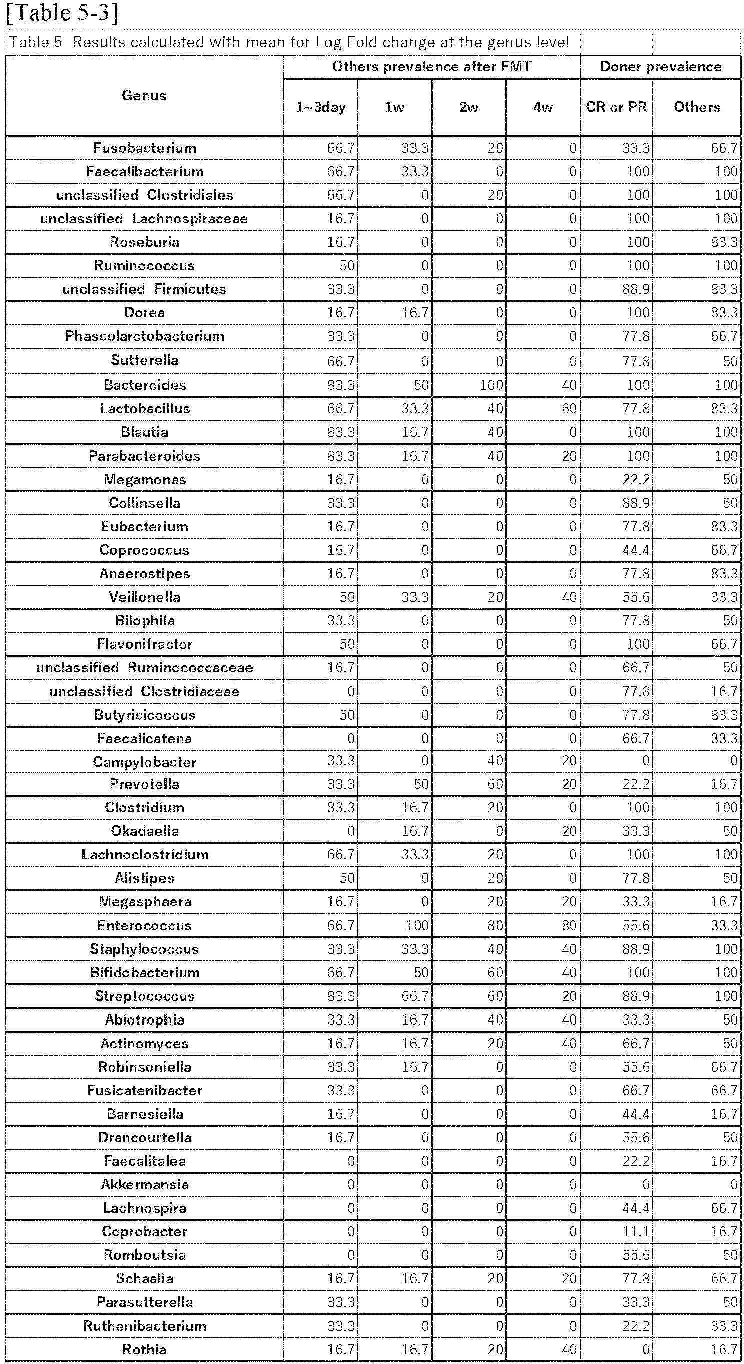

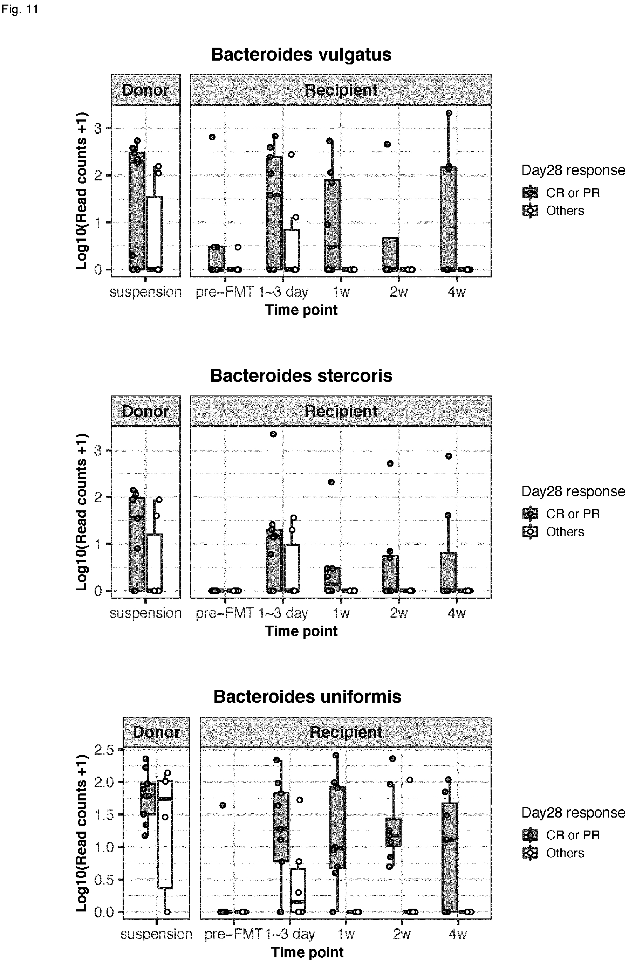

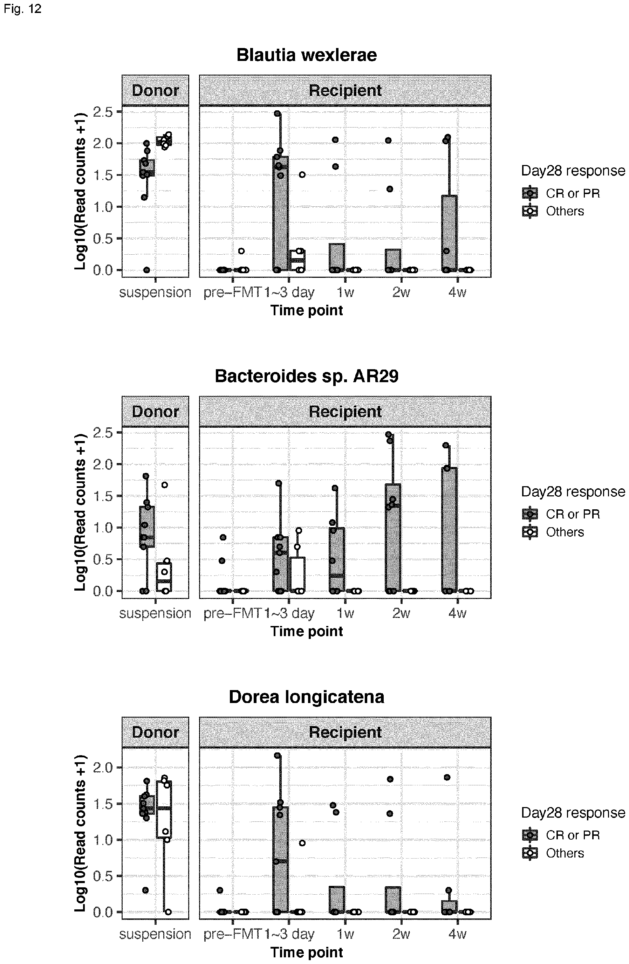

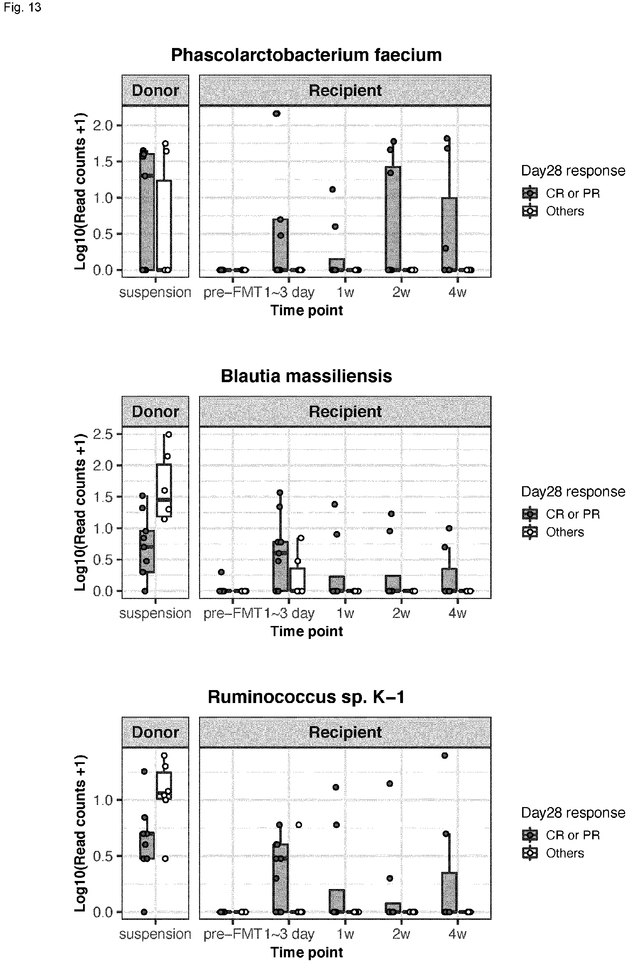

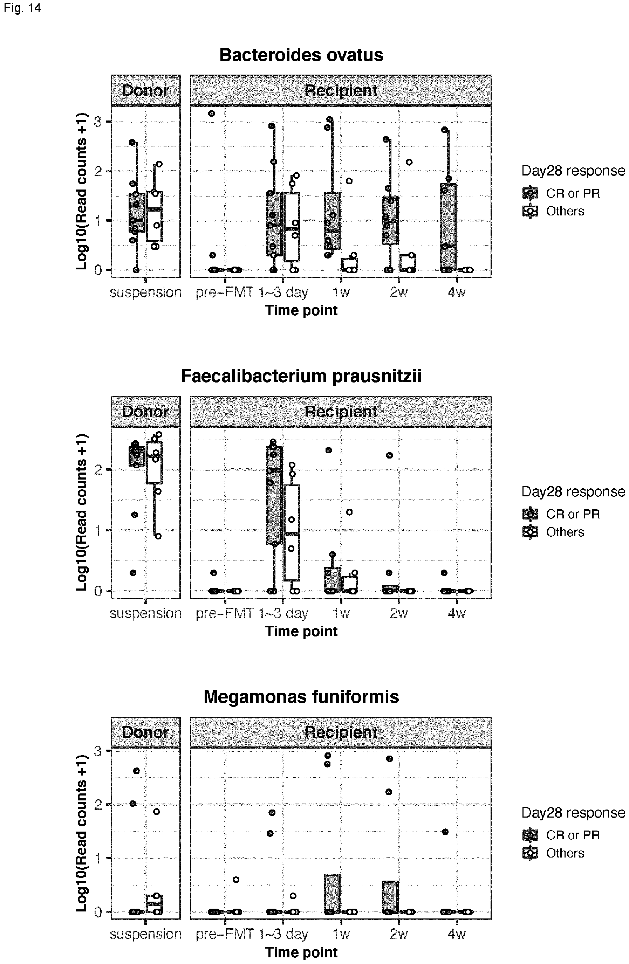

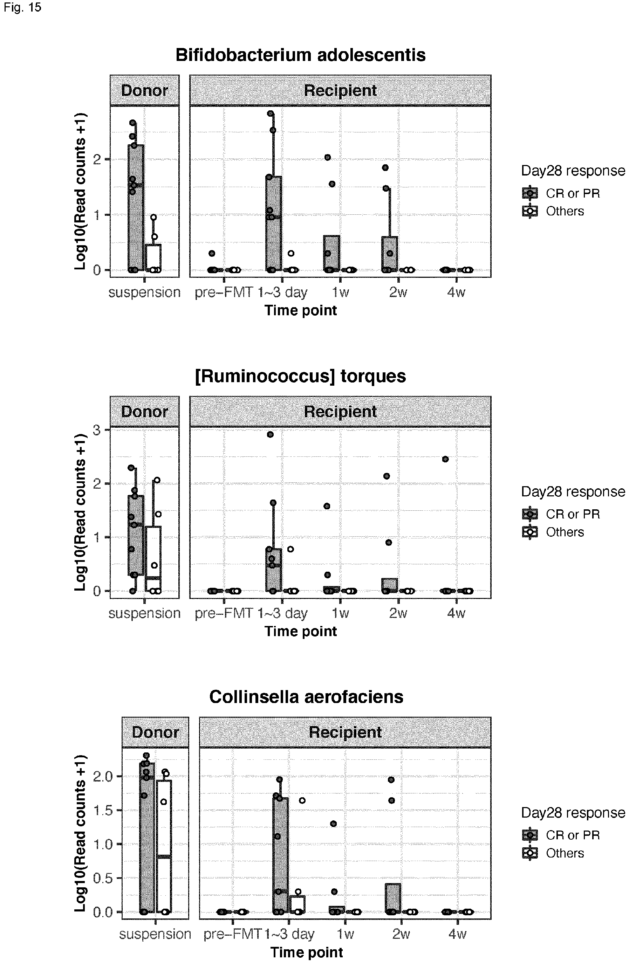

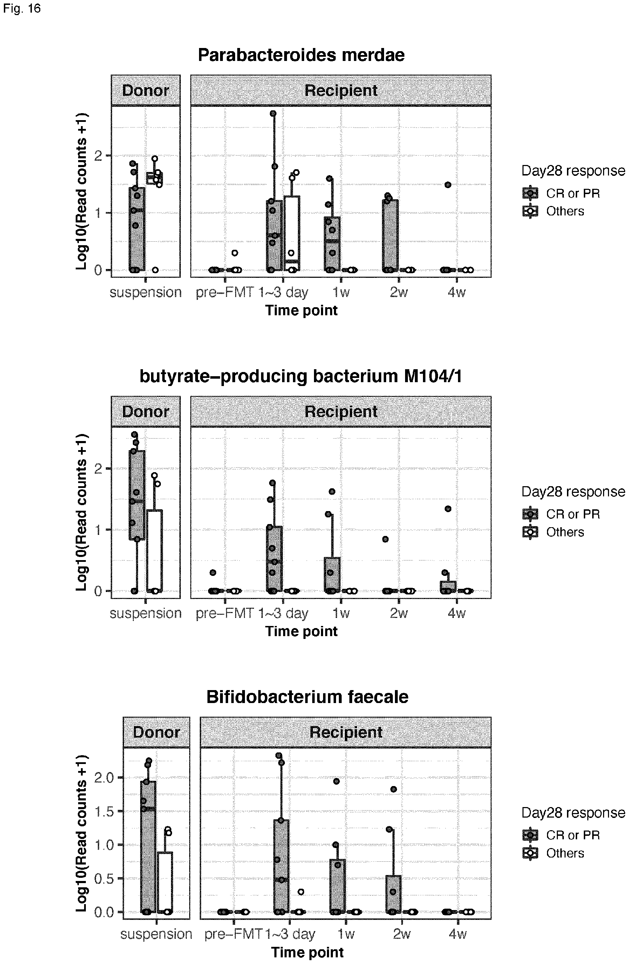

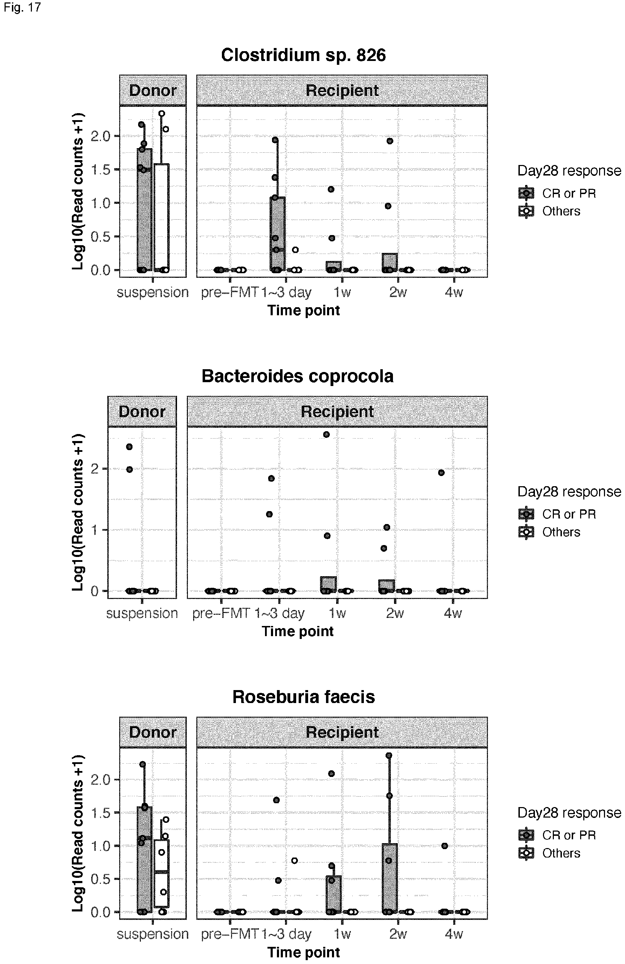

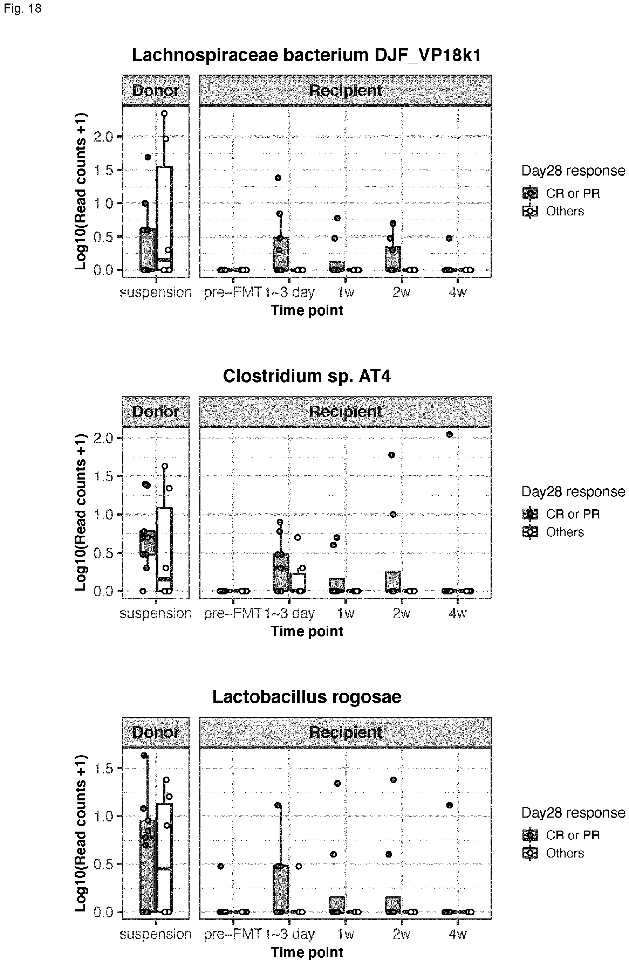

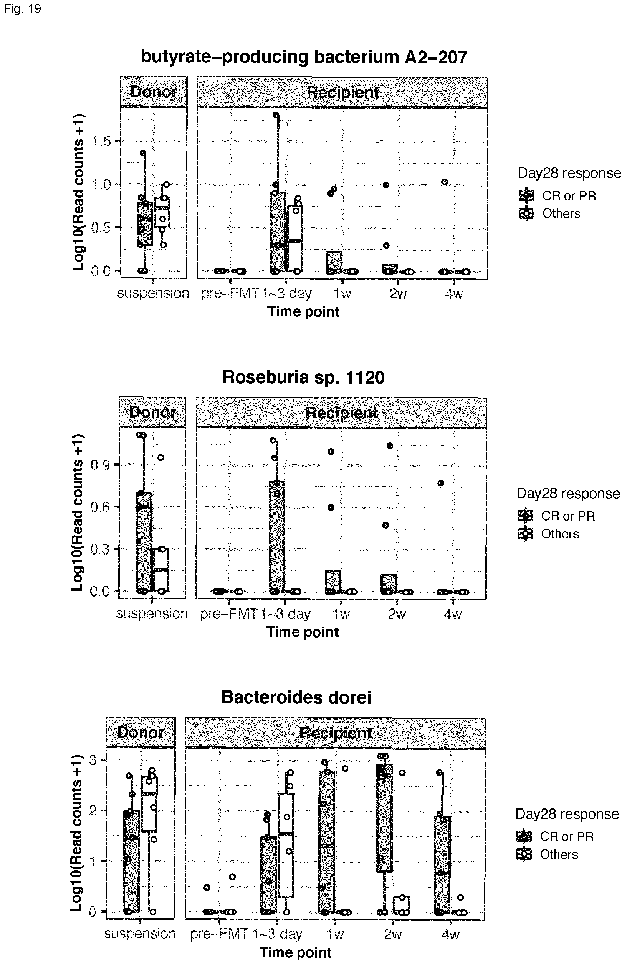

Further, among species in the fecal microbiota before and after FMT, the time courses of changes in the species of the above Group B are shown in Figures 11 to 19. The species shown in Figures 11 to 19 were all confirmed to be abundant in the CR or PR group in comparison with the Others group.

### Sequence Listing Free Text

SEQ ID NO: 345: synthetic DNA
SEQ ID NO: 346: synthetic DNA

## Claims

1. A prophylactic or therapeutic composition for GVHD, which comprises bacteria belonging to any genus selected from the group consisting of the following genera:
Blautia,
Clostridium,
unclassified Clostridiales,
Actinomyces,
Parabacteroides,
Lachnoclostridium,
Bacteroides,
Faecalibacterium,
unclassified Lachnospiraceae,
Roseburia,
Ruminococcus,
unclassified Firmicutes,
Dorea,
Phascolarctobacterium,
Sutterella,
Megamonas,
Collinsella,
Eubacterium,
Coprococcus,
Schaalia,
Alistipes,
Bifidobacterium,
Lactobacillus,
Veillonella,
Anaerostipes,
Bilophila,
Butyricicoccus,
Barnesiella,
Fusicatenibacter,
Flavonifractor,
unclassified Ruminococcaceae,
unclassified Clostridiaceae,
Faecalicatena,
Prevotella,
Megasphaera,
Robinsoniella,
Faecalitalea,
Lachnospira, and
Romboutsia,
or any combination of bacteria belonging to these genera.

2. The composition according to claim 1, which comprises bacteria belonging to any genus selected from the group consisting of the following genera:
Blautia,
Clostridium,
unclassified Clostridiales,
Actinomyces,
Parabacteroides,
Lachnoclostridium,
Bacteroides,
Faecalibacterium,
unclassified Lachnospiraceae,
Roseburia,
Ruminococcus,
unclassified Firmicutes,
Dorea,
Phascolarctobacterium,
Sutterella,
Megamonas,
Collinsella,
Eubacterium, and
Coprococcus,
or any combination of bacteria belonging to these genera.

3. The composition according to claim 1, which comprises bacteria belonging to any genus selected from the group consisting of the following genera:
Blautia,
Clostridium,
unclassified Clostridiales,
Actinomyces,
Parabacteroides,
Lachnoclostridium,
Bacteroides,
unclassified Lachnospiraceae,
Roseburia,
Ruminococcus,
Phascolarctobacterium,
Faecalibacterium,
Schaalia,
Alistipes,
Bifidobacterium,
Lactobacillus,
Veillonella,
Dorea,
unclassified Firmicutes,
Anaerostipes,
Collinsella,
Butyricicoccus,
Barnesiella, and
Fusicatenibacter,
or any combination of bacteria belonging to these genera.

4. The composition according to claim 3, which comprises bacteria belonging to any genus selected from the group consisting of the following genera:
Blautia,
Clostridium,
unclassified Clostridiales,
Actinomyces,
Parabacteroides,
Lachnoclostridium, and
Bacteroides,
or any combination of bacteria belonging to these genera.

5. The composition according to claim 1, which comprises bacteria belonging to any genus selected from the group consisting of the following genera:
Faecalibacterium,
unclassified Clostridiales,
unclassified Lachnospiraceae,
Roseburia,
Ruminococcus,
unclassified Firmicutes,
Dorea,
Phascolarctobacterium,
Sutterella,
Bacteroides,
Blautia,
Parabacteroides,
Megamonas,
Collinsella,
Eubacterium,
Coprococcus,
Anaerostipes,
Bilophila,
Flavonifractor,
unclassified Ruminococcaceae,
unclassified Clostridiaceae,
Butyricicoccus,
Faecalicatena,
Prevotella,
Clostridium,
Lachnoclostridium,
Alistipes,
Megasphaera,
Robinsoniella,
Fusicatenibacter,
Barnesiella,
Faecalitalea,
Lachnospira, and
Romboutsia,
or any combination of bacteria belonging to these genera.

6. The composition according to claim 5, which comprises bacteria belonging to any genus selected from the group consisting of the following genera:
Faecalibacterium,
unclassified Clostridiales,
unclassified Lachnospiraceae,
Roseburia,
Ruminococcus,
unclassified Firmicutes,
Dorea,
Phascolarctobacterium,
Sutterella,
Bacteroides,
Blautia,
Parabacteroides,
Megamonas,
Collinsella,
Eubacterium, and
Coprococcus,
or any combination of bacteria belonging to these genera.

7. A prophylactic or therapeutic composition for GVHD, which comprises bacteria comprising DNA consisting of a nucleotide sequence sharing 94% or more homology with any of the nucleotide sequences shown in SEQ ID NOs: 1 to 120, or any combination of these bacteria.

8. The composition according to claim 7, which comprises bacteria comprising DNA consisting of a nucleotide sequence sharing 94% or more homology with any of the nucleotide sequences shown in SEQ ID NOs: 121 to 152, or any combination of these bacteria.

9. The composition according to claim 7, which comprises bacteria comprising DNA consisting of a nucleotide sequence sharing 94% or more homology with any of the nucleotide sequences shown in SEQ ID NOs: 153 to 198, or any combination of these bacteria.

10. The composition according to claim 9, which comprises bacteria comprising DNA consisting of a nucleotide sequence sharing 94% or more homology with any of the nucleotide sequences shown in SEQ ID NOs: 199 to 201, or any combination of these bacteria.

11. The composition according to claim 7, which comprises bacteria comprising DNA consisting of a nucleotide sequence sharing 94% or more homology with any of the nucleotide sequences shown in SEQ ID NOs: 202 to 312, or any combination of these bacteria.

12. The composition according to claim 11, which comprises bacteria comprising DNA consisting of a nucleotide sequence sharing 94% or more homology with any of the nucleotide sequences shown in SEQ ID NOs: 313 to 344, or any combination of these bacteria.

13. The composition according to claim 1, wherein the bacteria comprise at least one selected from the group consisting of:
Bacteroides vulgatus,
Bacteroides stercoris,
Bacteroides uniformis,
Blautia wexlerae,
Bacteroides sp. AR29,
Dorea longicatena,
Phascolarctobacterium faecium,
Blautia massiliensis,
Ruminococcus sp. K-1,
Bacteroides ovatus,
Faecalibacterium prausnitzii,
Megamonas funiformis,
Bifidobacterium adolescentis,
[Ruminococcus] torques,
Collinsella aerofaciens,
Parabacteroides merdae,
butyrate-producing bacterium M104/1,
Bifidobacterium faecale,
Clostridium sp. 826,
Bacteroides coprocola,
Roseburia faecis,
Lachnospiraceae bacterium DJF_VP18k1,
Clostridium sp. AT4,
Lactobacillus rogosae,
butyrate-producing bacterium A2-207,
Roseburia sp. 1120,
Bacteroides dorei,
Bifidobacterium pseudocatenulatum,
Megasphaera massiliensis,
Bacteroides massiliensis,
Anaerostipes hadrus,
Alistipes putredinis,
Parabacteroides sp. D25,
Roseburia inulinivorans,
Bilophila sp. 4_1_30,
Flavonifractor plautii,
Eubacterium ventriosum,
Clostridiales bacterium 80/3,
Butyricicoccus faecihominis,
Bacteroides cellulosilyticus,
Coprococcus catus,
Parabacteroides j ohnsonii,
Lachnospiraceae bacterium DJF_RP14,
[Ruminococcus] gnavus,
Bifidobacterium longum,
Bacteroides xylanisolvens,
Prevotella stercorea,
Bacteroides plebeius,
Firmicutes bacterium DJF_VR50,
Sutterella wadsworthensis,
Lachnospiraceae bacterium 1_1_57FAA,
Blautia obeum,
Barnesiella intestinihominis,
[Eubacterium] eligens,
Coprococcus comes,
Ruminococcus sp. DJF _VR70k1,
butyrate-producing bacterium A2-175,
Faecalitalea cylindroides,
Blautia sp. YHC-4,
[Clostridium] glycyrrhizinilyticum,
Ruminococcus sp. 653,
Ruminococcus lactaris,
butyrate-producing bacterium SM6/1,
butyrate-producing bacterium SS3/4,
Clostridiaceae bacterium DJF_LS40,
Bacteroides sp. 1_1_30,
Bacteroides nordii,
Fusicatenibacter saccharivorans,
butyrate-producing bacterium SL7/1,
[Clostridium] scindens,
Parabacteroides distasonis,
Schaalia odontolytica,
Bacteroides faecis,
Bacteroides fragilis,
[Clostridium] bolteae, and
Clostridium sp. HGF2.

14. The composition according to claim 13, wherein the bacteria comprise at least one selected from the group consisting of:
Bacteroides vulgatus,
Bacteroides stercoris,
Bacteroides uniformis,
Blautia wexlerae,
Bacteroides sp. AR29,
Dorea longicatena,
Phascolarctobacterium faecium,
Blautia massiliensis,
Ruminococcus sp. K-1,
Bacteroides ovatus,
Faecalibacterium prausnitzii,
Megamonas funiformis,
Bifidobacterium adolescents,
[Ruminococcus] torques,
Collinsella aerofaciens,
Parabacteroides merdae,
butyrate-producing bacterium M104/1,
Bifidobacterium faecale,
Clostridium sp. 826,
Bacteroides coprocola,
Roseburia faecis,
Lachnospiraceae bacterium DJF_VP18k1,
Clostridium sp. AT4,
Lactobacillus rogosae,
butyrate-producing bacterium A2-207,
Roseburia sp. 1120, and
Bacteroides dorei.

15. The composition according to claim 13, wherein the bacteria comprise at least one selected from the group consisting of:
Bacteroides vulgatus,
Bacteroides stercoris,
Bacteroides uniformis,
Blautia wexlerae,
Bacteroides sp. AR29,
Dorea longicatena,
Phascolarctobacterium faecium,
Blautia massiliensis,
Ruminococcus sp. K-1, and
Bacteroides ovatus.

16. The composition according to any one of claims 1 to 15, wherein the GVHD is steroid-refractory or steroid-dependent GVHD

17. The composition according to any one of claims 1 to 15, wherein the GVHD is acute gut GVHD

18. A capsule formulation for the prevention or treatment of GVHD, which comprises the composition according to any one of claims 1 to 17.

19. A therapeutic method for GVHD, which comprises administering the composition according to any one of claims 1 to 17 or the capsule formulation according to claim 18 to a GVHD patient.

20. A prophylactic method for GVHD, which comprises administering the composition according to any one of claims 1 to 17 or the capsule formulation according to claim 18 either before or after or both before and after hematopoietic stem cell transplantation to a patient who is a subject of the hematopoietic stem cell transplantation.

21. A method for preparing a suspension for fecal microbiota transplantation, which comprises confirming the presence of the bacteria shown in any one of claims 1 to 15 in feces collected from a human subject, and suspending the feces confirmed for the presence of the bacteria into an aqueous medium.

22. A method for preparing a bacterial mixture for fecal microbiota transplantation, which comprises confirming the presence of the bacteria shown in any one of claims 1 to 15 in feces collected from a human subject, separating the bacteria from the feces confirmed for the presence of the bacteria, and mixing the separated bacteria.

23. A method for preparing a formulation for fecal microbiota transplantation, which comprises confirming the presence of the bacteria shown in any one of claims 1 to 15 in feces collected from a human subject, separating the bacteria from the feces confirmed for the presence of the bacteria, and formulating the separated bacteria.

24. The method according to claim 23, wherein the formulation is a capsule formulation.

25. The method according to any one of claims 21 to 24, wherein the presence of the bacteria is confirmed by 16S rRNA gene analysis.
